# EUROPEAN PATENT APPLICATION

(11) **EP 2 039 772 A2**
(43) Date of publication of application: **25.03.2009**
(21) Application number: 09150087.6
(22) Date of filing: 06.01.2009
(51) Int. Cl.: C12N 15/82, A01N 43/40

(54) **Method for improved utilization of the production potential of transgenic plants introduction**

(71) Applicant: Bayer CropScience AG, 40789 Monheim (DE)
(72) Inventor: The designation of the inventor has not yet been filed

(57) **Abstract**

The Invention discloses novel uses and methods for Fluopyram directed to increasing the production potential of transgenic plants as well as methods for controlling unwanted animal pests on transgenic plants by application of the compounds to the transgenic plants or to propagation material of these plants.

## Description

The invention relates to a method for improving the utilization of the production potential of transgenic plants.

In recent years, there has been a marked increase in the proportion of transgenic plants in agriculture, even if regional differences are still noticeable to date. Thus, for example, the proportion of transgenic maize in the USA has doubled from 26% to 52% since 2001, while transgenic maize has hardly been of any practical importance in Germany. However, in other European countries, for example in Spain, the proportion of transgenic maize is already about 12%.

Transgenic plants are employed mainly to utilize the production potential of respective plant varieties in the most favourable manner, at the lowest possible input of production means. The aim of the genetic modification of the plants is in particular the generation of resistance in the plants to certain pests or harmful organisms or else herbicides and also to abiotic stress (for example drought, heat or elevated salt levels). It is also possible to modify a plant genetically to increase certain quality or product features, such as, for example, the content of selected vitamins or oils, or to improve certain fibre properties.

Herbicide resistance or tolerance can be achieved, for example, by incorporating genes into the useful plant for expressing enzymes to detoxify certain herbicides, so that a relatively unimpeded growth of these plants is possible even in the presence of these herbicides for controlling broadleaved weeds and weed grasses. Examples which may be mentioned are cotton varieties or maize varieties which tolerate the herbicidally active compound glyphosate (Roundup®), (Roundup Ready®, Monsanto) or the herbicides glufosinate or oxynil.

More recently, there has also been the development of useful plants comprising two or more genetic modifications ("stacked transgenic plants" or multiply transgenic crops). Thus, for example, Monsanto has developed multiply transgenic maize varieties which are resistant to the European corn borer (*Ostrinia nubilalis*) and the Western corn rootworm (*Diabrotica virgifera*). Also known are maize and cotton crops which are both resistant to the Western corn rootworm and the cotton bollworm and tolerant to the herbicide Roundup®.

It has now been found that the utilization of the production potential of transgenic useful plants can be improved even more by treating the plants with N- {2-[3-chloro-5-(trifluoromethyl)-2-pyridinyl]ethyl}-2-trifluoromethylbenzamide (hereinafter "Fluopyram").

Fluopyram of the formula (I) 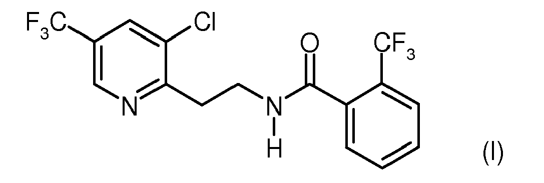 and its manufacturing process starting from known and commercially available compounds is described in WO 2004/016088.

Here, the term "treatment" includes all measures resulting in a contact between the active compound and at least one plant part. "Plant parts" are to be understood as meaning all above-ground and below-ground parts and organs of plants, such as shoot, leaf, flower and root, by way of example leaves, needles, stalks, stems, flowers, fruit bodies, fruits and seed, and also roots, tubers and rhizomes. The plant parts also include harvested material and also vegetative and generative propagation material, for example cuttings, tubers, rhizomes, slips and seed.

According to the invention all plants and plant parts can be treated. By plants is meant all plants and plant populations such as desirable and undesirable wild plants, cultivars and plant varieties (whether or not protectable by plant variety or plant breeder's rights). Cultivars and plant varieties can be plants obtained by conventional propagation and breeding methods which can be assisted or supplemented by one or more biotechnological methods such as by use of double haploids, protoplast fusion, random and directed mutagenesis, molecular or genetic markers or by bioengineering and genetic engineering methods. By plant parts is meant all above ground and below ground parts and organs of plants such as shoot, leaf, blossom and root, whereby for example leaves, needles, stems, branches, blossoms, fruiting bodies, fruits and seed as well as roots, corms and rhizomes are listed. Crops and vegetative and generative propagating material, for example cuttings, corms, rhizomes, runners and seeds also belong to plant parts.

Among the plants that can be protected by the method according to the invention, mention may be made of major field crops like corn, soybean, cotton, *Brassica* oilseeds such as *Brassica napus* (e.g. canola), *Brassica rapa, B. juncea* (e.g. mustard) and *Brassica carinata,* rice, wheat, sugarbeet, sugarcane, oats, rye, barley, millet, triticale, flax, vine and various fruits and vegetables of various botanical taxa such as *Rosaceae sp.* (for instance pip fruit such as apples and pears, but also stone fruit such as apricots, cherries, almonds and peaches, berry fruits such as strawberries), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (for instance banana trees and plantings), *Rubiaceae sp.* (for instance coffee), *Theaceae sp., Sterculiceae sp., Rutaceae sp.* (for instance lemons, oranges and grapefruit) ; *Solanaceae sp.* (for instance tomatoes, potatoes, peppers, eggplant), *Liliaceae sp.,* Compositiae sp. (for instance lettuce, artichoke and chicory - including root chicory, endive or common chicory), *Umbelliferae sp.* (for instance carrot, parsley, celery and celeriac), *Cucurbitaceae sp.* (for instance cucumber - including pickling cucumber, squash, watermelon, gourds and melons), *Alliaceae* sp. (for instance onions and leek), Cruciferae *sp.* (for instance white cabbage, red cabbage, broccoli, cauliflower, brussel sprouts, pak choi, kohlrabi, radish, horseradish, cress, Chinese cabbage), Leguminosae *sp.* (for instance peanuts, peas and beans beans - such as climbing beans and broad beans), *Chenopodiaceae sp.* (for instance mangold, spinach beet, spinach, beetroots), *Malvaceae* (for instance okra), *Asparagaceae* (for instance asparagus); horticultural and forest crops; ornamental plants; as well as genetically modified homologues of these crops.

The method of treatment according to the invention can be used in the treatment of genetically modified organisms (GMOs), e.g. plants or seeds. Genetically modified plants (or transgenic plants) are plants of which a heterologous gene has been stably integrated into genome. The expression "heterologous gene" essentially means a gene which is provided or assembled outside the plant and when introduced in the nuclear, chloroplastic or mitochondrial genome gives the transformed plant new or improved agronomic or other properties by expressing a protein or polypeptide of interest or by downregulating or silencing other gene(s) which are present in the plant (using for example, antisense technology, cosuppression technology or RNA interference - RNAi - technology). A heterologous gene that is located in the genome is also called a transgene. A transgene that is defined by its particular location in the plant genome is called a transformation or transgenic event.

Depending on the plant species or plant cultivars, their location and growth conditions (soils, climate, vegetation period, diet), the treatment according to the invention may also result in superadditive ("synergistic") effects. Thus, for example, reduced application rates and/or a widening of the activity spectrum and/or an increase in the activity of the active compounds and compositions which can be used according to the invention, better plant growth, increased tolerance to high or low temperatures, increased tolerance to drought or to water or soil salt content, increased flowering performance, easier harvesting, accelerated maturation, higher harvest yields, bigger fruits, larger plant height, greener leaf color, earlier flowering, higher quality and/or a higher nutritional value of the harvested products, higher sugar concentration within the fruits, better storage stability and/or processability of the harvested products are possible, which exceed the effects which were actually to be expected.

At certain application rates, the active compound combinations according to the invention may also have a strengthening effect in plants. Accordingly, they are also suitable for mobilizing the defense system of the plant against attack by unwanted microorganisms. This may, if appropriate, be one of the reasons of the enhanced activity of the combinations according to the invention, for example against fungi. Plant-strengthening (resistance-inducing) substances are to be understood as meaning, in the present context, those substances or combinations of substances which are capable of stimulating the defense system of plants in such a way that, when subsequently inoculated with unwanted microorganisms, the treated plants display a substantial degree of resistance to these microorganisms. In the present case, unwanted microorganisms are to be understood as meaning phytopathogenic fungi, bacteria and viruses. Thus, the substances according to the invention can be employed for protecting plants against attack by the abovementioned pathogens within a certain period of time after the treatment. The period of time within which protection is effected generally extends from 1 to 10 days, preferably 1 to 7 days, after the treatment of the plants with the active compounds.

Plants and plant cultivars which are preferably to be treated according to the invention include all plants which have genetic material which impart particularly advantageous, useful traits to these plants (whether obtained by breeding and/or biotechnological means).

Plants and plant cultivars which are also preferably to be treated according to the invention are resistant against one or more biotic stresses, i.e. said plants show a better defense against animal and microbial pests, such as against nematodes, insects, mites, phytopathogenic fungi, bacteria, viruses and/or viroids.

Plants and plant cultivars which may also be treated according to the invention are those plants which are resistant to one or more abiotic stresses. Abiotic stress conditions may include, for example, drought, cold temperature exposure, heat exposure, osmotic stress, flooding, increased soil salinity, increased mineral exposure, ozone exposure, high light exposure, limited availability of nitrogen nutrients, limited availability of phosphorus nutrients, shade avoidance.

Plants and plant cultivars which may also be treated according to the invention, are those plants characterized by enhanced yield characteristics. Increased yield in said plants can be the result of, for example, improved plant physiology, growth and development, such as water use efficiency, water retention efficiency, improved nitrogen use, enhanced carbon assimilation, improved photosynthesis, increased germination efficiency and accelerated maturation. Yield can furthermore be affected by improved plant architecture (under stress and non-stress conditions), including but not limited to, early flowering, flowering control for hybrid seed production, seedling vigor, plant size, internode number and distance, root growth, seed size, fruit size, pod size, pod or ear number, seed number per pod or ear, seed mass, enhanced seed filling, reduced seed dispersal, reduced pod dehiscence and lodging resistance. Further yield traits include seed composition, such as carbohydrate content, protein content, oil content and composition, nutritional value, reduction in anti-nutritional compounds, improved processability and better storage stability.

Examples of plants with the above-mentioned traits are non-exhaustively listed in Table A and B. Plants that may be treated according to the invention are hybrid plants that already express the characteristic of heterosis or hybrid vigor which results in generally higher yield, vigor, health and resistance towards biotic and abiotic stresses). Such plants are typically made by crossing an inbred male-sterile parent line (the female parent) with another inbred male-fertile parent line (the male parent). Hybrid seed is typically harvested from the male sterile plants and sold to growers. Male sterile plants can sometimes (e.g. in corn) be produced by detasseling, i.e. the mechanical removal of the male reproductive organs (or males flowers) but, more typically, male sterility is the result of genetic determinants in the plant genome. In that case, and especially when seed is the desired product to be harvested from the hybrid plants it is typically useful to ensure that male fertility in the hybrid plants is fully restored. This can be accomplished by ensuring that the male parents have appropriate fertility restorer genes which are capable of restoring the male fertility in hybrid plants that contain the genetic determinants responsible for male-sterility. Genetic determinants for male sterility may be located in the cytoplasm. Examples of cytoplasmic male sterility (CMS) were for instance described in Brassica species (W0 92/05251, WO 95/09910, WO 98/27806, WO 05/002324, WO 06/021972 and US 6,229,072). However, genetic determinants for male sterility can also be located in the nuclear genome. Male sterile plants can also be obtained by plant biotechnology methods such as genetic engineering. A particularly useful means of obtaining male-sterile plants is described in WO 89/10396 in which, for example, a ribonuclease such as barnase is selectively expressed in the tapetum cells in the stamens. Fertility can then be restored by expression in the tapetum cells of a ribonuclease inhibitor such as barstar (e.g. WO 91/02069).

Plants or plant cultivars (obtained by plant biotechnology methods such as genetic engineering) which may be treated according to the invention are herbicide-tolerant plants, i.e. plants made tolerant to one or more given herbicides. Such plants can be obtained either by genetic transformation, or by selection of plants containing a mutation imparting such herbicide tolerance.

Herbicide-resistant plants are for example glyphosate-tolerant plants, i.e. plants made tolerant to the herbicide glyphosate or salts thereof. Plants can be made tolerant to glyphosate through different means. For example, glyphosate-tolerant plants can be obtained by transforming the plant with a gene encoding the enzyme 5-enolpyruvylshikimate-3-phosphate synthase (EPSPS). Examples of such EPSPS genes are the AroA gene (mutant CT7) of the bacterium *Salmonella typhimurium* (Comai et al., 1983, Science 221, 370-371), the CP4 gene of the bacterium *Agrobacterium sp.* (Barry et al., 1992, Curr. Topics Plant Physiol. 7, 139-145), the genes encoding a Petunia EPSPS (Shah et al., 1986, Science 233, 478-481), a Tomato EPSPS (Gasser et al., 1988, J. Biol. Chem. 263, 4280-4289), or an Eleusine EPSPS (WO 01/66704). It can also be a mutated EPSPS as described in for example EP 0837944, WO 00/66746, WO 00/66747 or WO02/26995. Glyphosate-tolerant plants can also be obtained by expressing a gene that encodes a glyphosate oxido-reductase enzyme as described in U.S. Patent Nos. 5,776,760 and 5,463,175. Glyphosate-tolerant plants can also be obtained by expressing a gene that encodes a glyphosate acetyl transferase enzyme as described in for example WO 02/36782, WO 03/092360, WO 05/012515 and WO 07/024782. Glyphosate-tolerant plants can also be obtained by selecting plants containing naturally-occurring mutations of the above-mentioned genes, as described in for example WO 01/024615 or WO 03/013226.

Other herbicide resistant plants are for example plants that are made tolerant to herbicides inhibiting the enzyme glutamine synthase, such as bialaphos, phosphinothricin or glufosinate. Such plants can be obtained by expressing an enzyme detoxifying the herbicide or a mutant glutamine synthase enzyme that is resistant to inhibition. One such efficient detoxifying enzyme is an enzyme encoding a phosphinothricin acetyltransferase (such as the bar or pat protein from Streptomyces species). Plants expressing an exogenous phosphinothricin acetyltransferase are for example described in U.S. Patent Nos. 5,561,236; 5,648,477; 5,646,024; 5,273,894; 5,637,489; 5,276,268; 5,739,082; 5,908,810 and 7,112,665.

Further herbicide-tolerant plants are also plants that are made tolerant to the herbicides inhibiting the enzyme hydroxyphenylpyruvatedioxygenase (HPPD). Hydroxyphenylpyruvatedioxygenases are enzymes that catalyze the reaction in which para-hydroxyphenylpyruvate (HPP) is transformed into homogentisate. Plants tolerant to HPPD-inhibitors can be transformed with a gene encoding a naturally-occurring resistant HPPD enzyme, or a gene encoding a mutated HPPD enzyme as described in WO 96/38567, WO 99/24585 and WO 99/24586. Tolerance to HPPD-inhibitors can also be obtained by transforming plants with genes encoding certain enzymes enabling the formation of homogentisate despite the inhibition of the native HPPD enzyme by the HPPD-inhibitor. Such plants and genes are described in WO 99/34008 and WO 02/36787. Tolerance of plants to HPPD inhibitors can also be improved by transforming plants with a gene encoding an enzyme prephenate deshydrogenase in addition to a gene encoding an HPPD-tolerant enzyme, as described in WO 2004/024928.

Still further herbicide resistant plants are plants that are made tolerant to acetolactate synthase (ALS) inhibitors. Known ALS-inhibitors include, for example, sulfonylurea, imidazolinone, triazolopyrimidines, pryimidinyoxy(thio)benzoates, and/or sulfonylaminocarbonyltriazolinone herbicides. Different mutations in the ALS enzyme (also known as acetohydroxyacid synthase, AHAS) are known to confer tolerance to different herbicides and groups of herbicides, as described for example in Tranel and Wright (2002, Weed Science 50:700-712), but also, in U.S. Patent No. 5,605,011, 5,378,824, 5,141,870, and 5,013,659. The production of sulfonylurea-tolerant plants and imidazolinone-tolerant plants is described in U.S. Patent Nos. 5,605,011; 5,013,659; 5,141,870; 5,767,361; 5,731,180; 5,304,732; 4,761,373; 5,331,107; 5,928,937; and 5,378,824; and international publication WO 96/33270. Other imidazolinone-tolerant plants are also described in for example WO 2004/040012, WO 2004/106529, WO 2005/020673, WO 2005/093093, WO 2006/007373, WO 2006/015376, WO 2006/024351, and WO 2006/060634. Further sulfonylurea- and imidazolinone-tolerant plants are also described in for example WO 07/024782.

Other plants tolerant to imidazolinone and/or sulfonylurea can be obtained by induced mutagenesis, selection in cell cultures in the presence of the herbicide or mutation breeding as described for example for soybeans in U.S. Patent 5,084,082, for rice in WO 97/41218, for sugar beet in U.S. Patent 5,773,702 and WO 99/057965, for lettuce in U.S. Patent 5,198,599, or for sunflower in WO 01/065922.

Plants or plant cultivars (obtained by plant biotechnology methods such as genetic engineering) which may also be treated according to the invention are insect-resistant transgenic plants, i.e. plants made resistant to attack by certain target insects. Such plants can be obtained by genetic transformation, or by selection of plants containing a mutation imparting such insect resistance.

An "insect-resistant transgenic plant", as used herein, includes any plant containing at least one transgene comprising a coding sequence encoding:
1) an insecticidal crystal protein from *Bacillus thuringiensis* or an insecticidal portion thereof, such as the insecticidal crystal proteins listed by Crickmore et al. (1998, Microbiology and Molecular Biology Reviews, 62: 807-813), updated by Crickmore et al. (2005) at the *Bacillus thuringiensis* toxin nomenclature, online at: http://www.lifesci.sussex.ac.uk/Home/Neil_Crickmore/Bt/), or insecticidal portions thereof, e.g., proteins of the Cry protein classes Cry1Ab, Cry1Ac, Cry1B, Cry1C, Cry1D, Cry1F, Cry2Ab, Cry3Aa, or Cry3Bb or insecticidal portions thereof (e.g. EP 1999141 and WO 2007/107302); or
2) a crystal protein from *Bacillus thuringiensis* or a portion thereof which is insecticidal in the presence of a second other crystal protein from *Bacillus thuringiensis* or a portion thereof, such as the binary toxin made up of the Cry34 and Cry35 crystal proteins (Moellenbeck et al. 2001, Nat. Biotechnol. 19: 668-72; Schnepf et al. 2006, Applied Environm. Microbiol. 71, 1765-1774) or the binary toxin made up of the Cry1A or Cry1F proteins and the Cry2Aa or Cry2Ab or Cry2Ae proteins (US Patent Appl. No. 12/214,022 and EP 08010791.5); or
3) a hybrid insecticidal protein comprising parts of different insecticidal crystal proteins from *Bacillus thuringiensis,* such as a hybrid of the proteins of 1) above or a hybrid of the proteins of 2) above, e.g., the Cry1A.105 protein produced by corn event MON89034 (WO 2007/027777); or
4) a protein of any one of 1) to 3) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation, such as the Cry3Bb1 protein in corn events MON863 or MON88017, or the Cry3A protein in corn event MIR604; or
5) an insecticidal secreted protein from *Bacillus thuringiensis* or *Bacillus cereus,* or an insecticidal portion thereof, such as the vegetative insecticidal (VIP) proteins listed at: http://www.lifesci.sussex.ac.uk/home/Neil CrickmoreBt/vip.html, e.g., proteins from the VIP3Aa protein class; or
6) a secreted protein from *Bacillus thuringiensis* or *Bacillus cereus* which is insecticidal in the presence of a second secreted protein from *Bacillus thuringiensis* or *B. cereus,* such as the binary toxin made up of the VIP1A and VIP2A proteins (WO 94/21795); or
7) a hybrid insecticidal protein comprising parts from different secreted proteins from *Bacillus thuringiensis* or *Bacillus cereus,* such as a hybrid of the proteins in 1) above or a hybrid of the proteins in 2) above; or
8) a protein of any one of 5) to 7) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation (while still encoding an insecticidal protein), such as the VIP3Aa protein in cotton event COT102; or
9) a secreted protein from *Bacillus thuringiensis* or *Bacillus cereus* which is insecticidal in the presence of a crystal protein from *Bacillus thuringiensis,* such as the binary toxin made up of VIP3 and Cry1A or Cry1F (US Patent Appl. No. 61/126083 and 61/195019), or the binary toxin made up of the VIP3 protein and the Cry2Aa or Cry2Ab or Cry2Ae proteins (US Patent Appl. No. 12/214,022 and EP 08010791.5).
10) a protein of 9) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation (while still encoding an insecticidal protein) Of course, an insect-resistant transgenic plant, as used herein, also includes any plant comprising a combination of genes encoding the proteins of any one of the above classes 1 to 10. In one embodiment, an insect-resistant plant contains more than one transgene encoding a protein of any one of the above classes 1 to 10, to expand the range of target insect species affected when using different proteins directed at different target insect species, or to delay insect resistance development to the plants by using different proteins insecticidal to the same target insect species but having a different mode of action, such as binding to different receptor binding sites in the insect.

An "insect-resistant transgenic plant", as used herein, further includes any plant containing at least one transgene comprising a sequence producing upon expression a double-stranded RNA which upon ingestion by a plant insect pest inhibits the growth of this insect pest, as described e.g. in WO 2007/080126.

Plants or plant cultivars (obtained by plant biotechnology methods such as genetic engineering) which may also be treated according to the invention are tolerant to abiotic stresses. Such plants can be obtained by genetic transformation, or by selection of plants containing a mutation imparting such stress resistance. Particularly useful stress tolerance plants include:
1) plants which contain a transgene capable of reducing the expression and/or the activity of poly(ADP-ribose) polymerase (PARP) gene in the plant cells or plants as described in WO 00/04173, WO/2006/045633, EP 04077984.5, or EP 06009836.5.
2) plants which contain a stress tolerance enhancing transgene capable of reducing the expression and/or the activity of the PARG encoding genes of the plants or plants cells, as described e.g. in WO 2004/090140.
3) plants which contain a stress tolerance enhancing transgene coding for a plant-functional enzyme of the nicotine amide adenine dinucleotide salvage synthesis pathway including nicotinamidase, nicotinate phosphoribosyltransferase, nicotinic acid mononucleotide adenyl transferase, nicotinamide adenine dinucleotide synthetase or nicotine amide phosphorybosyltransferase as described e.g. in EP 04077624.7, WO 2006/133827, PCT/EP07/002433, EP 1999263, or WO 2007/107326.

Plants or plant cultivars (obtained by plant biotechnology methods such as genetic engineering) which may also be treated according to the invention show altered quantity, quality and/or storage-stability of the harvested product and/or altered properties of specific ingredients of the harvested product such as :
1) transgenic plants which synthesize a modified starch, which in its physical-chemical characteristics, in particular the amylose content or the amylose/amylopectin ratio, the degree of branching, the average chain length, the side chain distribution, the viscosity behaviour, the gelling strength, the starch grain size and/or the starch grain morphology, is changed in comparison with the synthesised starch in wild type plant cells or plants, so that this is better suited for special applications. Said transgenic plants synthesizing a modified starch are disclosed, for example, in EP 0571427, WO 95/04826, EP 0719338, WO 96/15248, WO 96/19581, WO 96/27674, WO 97/11188, WO 97/26362, WO 97/32985, WO 97/42328, WO 97/44472, WO 97/45545, WO 98/27212, WO 98/40503, WO99/58688, WO 99/58690, WO 99/58654, WO 00/08184, WO 00/08185, WO 00/08175, WO 00/28052, WO 00/77229, WO 01/12782, WO 01/12826, WO 02/101059, WO 03/071860, WO 2004/056999, WO 2005/030942, WO 2005/030941, WO 2005/095632, WO 2005/095617, WO 2005/095619, WO 2005/095618, WO 2005/123927, WO 2006/018319, WO 2006/103107, WO 2006/108702, WO 2007/009823, WO 00/22140, WO 2006/063862, WO 2006/072603, WO 02/034923, EP 06090134.5, EP 06090228.5, EP 06090227.7, EP 07090007.1, EP 07090009.7, WO 01/14569, WO 02/79410, WO 03/33540, WO 2004/078983, WO 01/19975, WO 95/26407, WO 96/34968, WO 98/20145, WO 99/12950, WO 99/66050, WO 99/53072, US 6,734,341, WO 00/11192, WO 98/22604, WO 98/32326, WO 01/98509, WO 01/98509, WO 2005/002359, US 5,824,790, US 6,013,861, WO 94/04693, WO 94/09144, WO 94/11520, WO 95/35026, WO 97/20936
2) transgenic plants which synthesize non starch carbohydrate polymers or which synthesize non starch carbohydrate polymers with altered properties in comparison to wild type plants without genetic modification. Examples are plants producing polyfructose, especially of the inulin and levan-type, as disclosed in EP 0663956, WO 96/01904, WO 96/21023, WO 98/39460, and WO 99/24593, plants producing alpha-1,4-glucans as disclosed in WO 95/31553, US 2002031826, US 6,284,479, US 5,712,107, WO 97/47806, WO 97/47807, WO 97/47808 and WO 00/14249, plants producing alpha-1,6 branched alpha-1,4-glucans, as disclosed in WO 00/73422, plants producing alternan, as disclosed in e.g. WO 00/47727, WO 00/73422, EP 06077301.7, US 5,908,975 and EP 0728213,
3) transgenic plants which produce hyaluronan, as for example disclosed in WO 2006/032538, WO 2007/039314, WO 2007/039315, WO 2007/039316, JP 2006304779, and WO 2005/012529.
4) transgenic plants or hybrid plants, such as onions with characteristics such as 'high soluble solids content', 'low pungency' (LP) and/or 'long storage' (LS), as described in US Patent Appl. No. 12/020,360 and 61/054,026.

Plants or plant cultivars (that can be obtained by plant biotechnology methods such as genetic engineering) which may also be treated according to the invention are plants, such as cotton plants, with altered fiber characteristics. Such plants can be obtained by genetic transformation, or by selection of plants contain a mutation imparting such altered fiber characteristics and include:
a) Plants, such as cotton plants, containing an altered form of cellulose synthase genes as described in WO 98/00549
b) Plants, such as cotton plants, containing an altered form of rsw2 or rsw3 homologous nucleic acids as described in WO 2004/053219
c) Plants, such as cotton plants, with increased expression of sucrose phosphate synthase as described in WO 01/17333
d) Plants, such as cotton plants, with increased expression of sucrose synthase as described in WO 02/45485
e) Plants, such as cotton plants, wherein the timing of the plasmodesmatal gating at the basis of the fiber cell is altered, e.g. through downregulation of fiber-selective β-1,3-glucanase as described in WO 2005/017157, or as described in EP 08075514.3 or US Patent Appl. No. 61/128,938
f) Plants, such as cotton plants, having fibers with altered reactivity, e.g. through the expression of N-acetylglucosaminetransferase gene including nodC and chitin synthase genes as described in WO 2006/136351

Plants or plant cultivars (that can be obtained by plant biotechnology methods such as genetic engineering) which may also be treated according to the invention are plants, such as oilseed rape or related Brassica plants, with altered oil profile characteristics. Such plants can be obtained by genetic transformation, or by selection of plants contain a mutation imparting such altered oil profile characteristics and include:
a) Plants, such as oilseed rape plants, producing oil having a high oleic acid content as described e.g. in US 5,969,169, US 5,840,946 or US 6,323,392 or US 6,063,947
b) Plants such as oilseed rape plants, producing oil having a low linolenic acid content as described in US 6,270,828, US 6,169,190 or US 5,965,755
c) Plant such as oilseed rape plants, producing oil having a low level of saturated fatty acids as described e.g. in US Patent No. 5,434,283 Plants or plant cultivars (that can be obtained by plant biotechnology methods such as genetic engineering) which may also be treated according to the invention are plants, such as oilseed rape or related Brassica plants, with altered seed shattering characteristics. Such plants can be obtained by genetic transformation, or by selection of plants contain a mutation imparting such altered seed shattering characteristics and include plants such as oilseed rape plants with delayed or reduced seed shattering as described in US Patent Appl. No. 61/135,230 and EP 08075648.9.

Particularly useful transgenic plants which may be treated according to the invention are plants containing transformation events, or combination of transformation events, that are the subject of petitions for non-regulated status, in the United States of America, to the Animal and Plant Health Inspection Service (APHIS) of the United States Department of Agriculture (USDA) whether such petitions are granted or are still pending. At any time this information is readily available from APHIS (4700 River Road Riverdale, MD 20737, USA), for instance on its internet site (URL http://www.aphis.usda.gov/brs/not_reg.html). On the filing date of this application the petitions for nonregulated status that were pending with APHIS or granted by APHIS were those listed in table B which contains the following information:
- Petition : the identification number of the petition. Technical descriptions of the transformation events can be found in the individual petition documents which are obtainable from APHIS, for example on the APHIS website, by reference to this petition number. These descriptions are herein incorporated by reference.
- Extension of Petition : reference to a previous petition for which an extension is requested.
- Institution : the name of the entity submitting the petition.
- Regulated article : the plant species concerned.
- Transgenic phenotype : the trait conferred to the plants by the transformation event.
- Transformation event or line : the name of the event or events (sometimes also designated as lines or lines) for which nonregulated status is requested.
- APHIS documents : various documents published by APHIS in relation to the Petition and which can be requested with APHIS.

Additional particularly useful plants containing single transformation events or combinations of transformation events are listed for example in the databases from various national or regional regulatory agencies (see for example http://gmoinfo.jrc.it/gmp browse.aspx and http://www.agbios.com/dbase.php).

Further particularly transgenic plants include plants containing a transgene in an agronomically neutral or beneficial position as described in any of the patent publications listed in Table C.

In a particularly preferred variant, the process according to the invention is used for treating transgenic vegetable, maize, soya bean, cotton, tobacco, rice, potato and sugar beet varieties. These are preferably plants that comprise Bt toxins.

The vegetable plants or varieties are, for example, the following useful plants:
○ potatoes: preferably starch potatoes, sweet potatoes and table potatoes;
○ root vegetables: preferably carrots, turnips (swedes, stubble turnips (*Brassica rapa* var. rapa), spring turnips, autumn turnips (*Brassica campestris* ssp. rapifera), *Brassica rapa* L. ssp. rapa f. teltowiensis), scorzonera, Jerusalem artichoke, turnip-rooted parsley, parsnip, radish and horseradish;
○ tuber vegetables: preferably kohlrabi, beetroot, celeriac, garden radish;
○ bulb crops: preferably scallion, leek and onions (planting onions and seed onions);
○ brassica vegetables: preferably headed cabbage (white cabbage, red cabbage, kale, savoy cabbage), cauliflowers, broccoli, curly kale, marrow-stem kale, seakale and Brussels sprouts;
○ fruiting vegetables: preferably tomatoes (outdoor tomatoes, vine-ripened tomatoes, beef tomatoes, greenhouse tomatoes, cocktail tomatoes, industrial and fresh market tomatoes), melons, eggplants, aubergines, pepper (sweet pepper and hot pepper, Spanish pepper), chilli pepper, pumpkins, courgettes and cucumbers (outdoor cucumbers, greenhouse cucumbers snake gourds and gherkins);
○ vegetable pulses: preferably bush beans (as sword beans, string beans, flageolet beans, wax beans, corn beans of green- and yellow-podded cultivars), pole beans (as sword beans, string beans, flageolet beans, wax beans of green-, blue- and yellow-podded cultivars), broadbeans (field beans, Windsor beans, cultivars having white- and black-spotted flowers), peas (chickling vetch, chickpeas, marrow peas, shelling peas, sugar-peas, smooth peas, cultivars having light- and dark-green fresh fruits) and lentils;
○ green vegetables and stem vegetables: preferably Chinese cabbage, round-headed garden lettuce, curled lettuce, lamb's-lettuce, iceberg lettuce, romaine lettuce, oakleaf lettuce, endives, radicchio, lollo rossa, ruccola lettuce, chicory, spinach, chard (leaf chard and stem chard) and parsley;
○ other vegetables: preferably asparagus, rhubarb, chives, artichokes, mint varieties, sunflowers, Florence fennel, dill, garden cress, mustard, poppy seed, peanuts, sesame und salad chicory.

Preferred embodiments of the invention are those treatments with Fluopyram wherein the transgenic plant:
a.) is selected from the plants listed in Table A: A-1 to A-131 or Table B: B-1 to B-85, or
b.) comprises one or more transgenic events selected from the transgenic events listed in Table A from A-1 to A-131 or in Table B from B-1 to B-85, or
c.) displays a trait based one or several transgenic events as listed in Table C from C-1 to C-11, or
d.) comprises a transgenic event selected from Table D from D-1 to D-48.

In a preferred embodiment of the invention, the transgenic plants are treated with Fluopyram to obtain a synergistic increase in
(i) the fungicidal efficacy and/or
(ii) the spectrum of activity against harmful pests and/or
(iii) the control of pests which display a partial or complete resistance or tolerance against the Fluopyram or the plant to be engineered to be resistant against wildtype or sensitive strains of foresaid pest.

The methods to determine the resistance of pests against active ingredients are well known to the person of ordinary skill in the art. Such methods can e.g. be found on the website of the "Insecticide Resistance Action Committee" under http://www.irac-online.org.

In a further preferred embodiment of the invention, the treatment of a transgenic plant with Fluopyram results in an increased yield of the transgenic plant, wherein the transgenic plant:
a.) is selected from the plants listed in Table A: A-1 to A-131 or Table B: B-1 to B-85, or
b.) comprises of one or more transgenic events selected from the transgenic events listed in Table A from A-1 to A-131 or in Table B from B-1 to B-85, or
c.) displays a trait based one or several transgenic events as listed in Table C from C-1 to C-11, or
d.) comprises a transgenic event selected from Table D from D-1 to D-48.

### Table A:

Non-exhaustive list of transgenic plants and events for working the invention. Source: AgBios database (AGBIOS, P.O. Box 475, 106 St. John St. Merrickville, Ontario K0G1N0, CANADA) which can be accessed under: http://www.agbios.com/dbase.php

| **No.** | **Transgenic event** | **Company** | **Description** | **Crop** |
|---|---|---|---|---|
| A-1 | ASR368 | Scotts Seeds | Glyphosate tolerance derived by | *Agrostis* |
| | | | inserting a modified 5- | *stolonifera* |
| | | | enolpyruvylshikimate-3-phosphate | *Creeping* |
| | | | synthase (EPSPS) encoding gene from | *Bentgrass* |
| | | | Agrobacterium tumefaciens. | |
| A-2 | H7-1 | Monsanto | Glyphosate herbicide tolerant sugar | *Beta vulgaris* |
| | | Company | beet produced by inserting a gene | |
| | | | encoding the enzyme 5- | |
| | | | enolypyruvylshikimate-3-phosphate | |
| | | | synthase (EPSPS) from the CP4 strain | |
| | | | of Agrobacterium tumefaciens. | |
| A-3 | T120-7 | Bayer | Introduction of the PPT- | *Beta vulgaris* |
| | | CropScience | acetyltransferase (PAT) encoding gene | |
| | | (Aventis | from Streptomyces viridochromogenes, | |
| | | CropScience(A | an aerobic soil bacteria. PPT normally | |
| | | grEvo)) | acts to inhibit glutamine synthetase, | |
| | | | causing a fatal accumulation of | |
| | | | ammonia. Acetylated PPT is inactive. | |
| A-4 | GTSB77 | Novartis | Glyphosate herbicide tolerant sugar | *Beta vulgaris* |
| | | Seeds; | beet produced by inserting a gene | *sugar Beet* |
| | | Monsanto | encoding the enzyme 5- | |
| | | Company | enolypyruvylshikimate-3-phosphate | |
| | | | synthase (EPSPS) from the CP4 strain | |
| | | | of Agrobacterium tumefaciens. | |
| A-5 | 23-18-17, | Monsanto | High laurate (12:0) and myristate | *Brassica* |
| | 23-198 | Company | (14:0) canola produced by inserting a | *napus* (Argenti |
| | | (formerly | thioesterase encoding gene from the | ne Canola) |
| | | Calgene) | California bay laurel (Umbellularia | |
| | | | californica). | |
| A-6 | 45A37, | Pioneer Hi- | High oleic acid and low linolenic acid | *Brassica* |
| | 46A40 | Bred | canola produced through a combination | *napus* (Argenti |
| | | International | of chemical mutagenesis to select for a | ne Canola) |
| | | Inc. | fatty acid desaturase mutant with | |
| | | | elevated oleic acid, and traditional | |
| | | | back-crossing to introduce the low | |
| | | | linolenic acid trait. | |
| A-7 | 46A12, | Pioneer Hi- | Combination of chemical mutagenesis, | *Brassica* |
| | 46A16 | Bred | to achieve the high oleic acid trait, and | *napus* (Argenti |
| | | International | traditional breeding with registered | ne Canola) |
| | | Inc. | canola varieties. | |
| A-8 | GT200 | Monsanto | Glyphosate herbicide tolerant canola | *Brassica* |
| | | Company | produced by inserting genes encoding | *napus* (Argenti |
| | | | the enzymes 5-enolypyruvylshikimate- | ne Canola) |
| | | | 3-phosphate synthase (EPSPS) from | |
| | | | the CP4 strain of Agrobacterium | |
| | | | tumefaciens and glyphosate oxidase | |
| | | | from Ochrobactrum anthropi. | |
| A-9 | GT73, | Monsanto | Glyphosate herbicide tolerant canola | *Brassica* |
| | RT73 | Company | produced by inserting genes encoding | *napus* (Argenti |
| | | | the enzymes 5-enolypyruvylshikimate- | ne Canola) |
| | | | 3-phosphate synthase (EPSPS) from | |
| | | | the CP4 strain of Agrobacterium | |
| | | | tumefaciens and glyphosate oxidase | |
| | | | from Ochrobactrum anthropi. | |
| A-10 | HCN10 | Aventis | Introduction of the PPT- | *Brassica* |
| | | CropScience | acetyltransferase (PAT) encoding gene | *napus* (Argenti |
| | | | from Streptomyces viridochromogenes, | ne Canola) |
| | | | an aerobic soil bacteria. PPT normally | |
| | | | acts to inhibit glutamine synthetase, | |
| | | | causing a fatal accumulation of | |
| | | | ammonia. Acetylated PPT is inactive. | |
| A-11 | HCN92 | Bayer | Introduction of the PPT- | *Brassica* |
| | | CropScience | acetyltransferase (PAT) encoding gene | *napus* (Argenti |
| | | (Aventis | from Streptomyces viridochromogenes, | ne Canola) |
| | | CropScience(A | an aerobic soil bacteria. PPT normally | |
| | | grEvo)) | acts to inhibit glutamine synthetase, | |
| | | | causing a fatal accumulation of | |
| | | | ammonia. Acetylated PPT is inactive. | |
| A-12 | MS1, RF1 | Aventis | Male-sterility, fertility restoration, | *Brassica* |
| | =>PGS1 | CropScience | pollination control system displaying | *napus* (Argenti |
| | | (formerly Plant | glufosinate herbicide tolerance. MS | ne Canola) |
| | | Genetic | lines contained the barnase gene from | |
| | | Systems) | Bacillus amyloliquefaciens, RF lines | |
| | | | contained the barstar gene from the | |
| | | | same bacteria, and both lines contained | |
| | | | the phosphinothricin N- | |
| | | | acetyltransferase (PAT) encoding gene | |
| | | | from Streptomyces hygroscopicus. | |
| A-13 | MS1, RF2 | Aventis | Male-sterility, fertility restoration, | *Brassica* |
| | =>PGS2 | CropScience | pollination control system displaying | *napus* (Argenti |
| | | (formerly Plant | glufosinate herbicide tolerance. MS | ne Canola) |
| | | Genetic | lines contained the barnase gene from | |
| | | Systems) | Bacillus amyloliquefaciens, RF lines | |
| | | | contained the barstar gene from the | |
| | | | same bacteria, and both lines contained | |
| | | | the phosphinothricin N- | |
| | | | acetyltransferase (PAT) encoding gene | |
| | | | from Streptomyces hygroscopicus. | |
| A-14 | MS8xRF3 | Bayer | Male-sterility, fertility restoration, | *Brassica* |
| | | CropScience | pollination control system displaying | *napus* (Argenti |
| | | (Aventis | glufosinate herbicide tolerance. MS | ne Canola) |
| | | CropScience(A | lines contained the barnase gene from | |
| | | grEvo)) | Bacillus amyloliquefaciens, RF lines | |
| | | | contained the barstar gene from the | |
| | | | same bacteria, and both lines contained | |
| | | | the phosphinothricin N- | |
| | | | acetyltransferase (PAT) encoding gene | |
| | | | from Streptomyces hygroscopicus. | |
| A-15 | NS738, | Pioneer Hi- | Selection of somaclonal variants with | *Brassica* |
| | NS1471, | Bred | altered acetolactate synthase (ALS) | *napus* (Argenti |
| | NS1473 | International | enzymes, following chemical | ne Canola) |
| | | Inc. | mutagenesis. Two lines (P1,P2) were | |
| | | | initially selected with modifications at | |
| | | | different unlinked loci. NS738 contains | |
| | | | the P2 mutation only. | |
| A-16 | OXY-235 | Aventis | Tolerance to the herbicides bromoxynil | *Brassica* |
| | | CropScience | and ioxynil by incorporation of the | *napus* (Argenti |
| | | (formerly | nitrilase gene from Klebsiella | ne Canola) |
| | | Rhône Poulenc | pneumoniae. | |
| | | Inc.) | | |
| A-17 | PHY14, | Aventis | Male sterility was via insertion of the | *Brassica* |
| | PHY35 | CropScience | barnase ribonuclease gene from | *napus* (Argenti |
| | | (formerly Plant | Bacillus amyloliquefaciens; fertility | ne Canola) |
| | | Genetic | restoration by insertion of the barstar | |
| | | Systems) | RNase inhibitor; PPT resistance was | |
| | | | via PPT-acetyltransferase (PAT) from | |
| | | | Streptomyces hygroscopicus. | |
| A-18 | PHY36 | Aventis | Male sterility was via insertion of the | *Brassica* |
| | | CropScience | barnase ribonuclease gene from | *napus* (Argenti |
| | | (formerly Plant | Bacillus amyloliquefaciens; fertility | ne Canola) |
| | | Genetic | restoration by insertion of the barstar | |
| | | Systems) | RNase inhibitor; PPT resistance was | |
| | | | via PPT-acetyltransferase (PAT) from | |
| | | | Streptomyces hygroscopicus. | |
| A-19 | T45 | Bayer | Introduction of the PPT- | *Brassica* |
| | (HCN28) | CropScience | acetyltransferase (PAT) encoding gene | *napus* (Argenti |
| | | (Aventis | from Streptomyces viridochromogenes, | ne Canola) |
| | | CropScience(A | an aerobic soil bacteria. PPT normally | |
| | | grEvo)) | acts to inhibit glutamine synthetase, | |
| | | | causing a fatal accumulation of | |
| | | | ammonia. Acetylated PPT is inactive. | |
| A-20 | HCR-1 | Bayer | Introduction of the glufosinate | *Brassica* |
| | | CropScience | ammonium herbicide tolerance trait | *rapa* (Polish |
| | | (Aventis | from transgenic B. napus line T45. This | Canola) |
| | | CropScience(A | trait is mediated by the | |
| | | grEvo)) | phosphinothricin acetyltransferase | |
| | | | (PAT) encoding gene from S. | |
| | | | viridochromogenes. | |
| A-21 | ZSR500/50 | Monsanto | Introduction of a modified 5-enol- | *Brassica* |
| | 2 | Company | pyruvylshikimate-3-phosphate synthase | *rapa* (Polish |
| | | | (EPSPS) and a gene from | Canola) |
| | | | Achromobacter sp that degrades | |
| | | | glyphosate by conversion to | |
| | | | aminomethylphosphonic acid (AMPA) | |
| | | | and glyoxylate by interspecific crossing | |
| | | | with GT73. | |
| A-22 | 55-1/63-1 | Cornell | Papaya ringspot virus (PRSV) resistant | *Carica* |
| | | University | papaya produced by inserting the coat | *papaya* (Papay |
| | | | protein (CP) encoding sequences from | a) |
| | | | this plant potyvirus. | |
| A-23 | RM3-3, | Bejo Zaden BV | Male sterility was via insertion of the | *Cichorium* |
| | RM3-4, | | barnase ribonuclease gene from | *intybus* (Chicor |
| | RM3-6 | | Bacillus amyloliquefaciens; PPT | y) |
| | | | resistance was via the bar gene from S. | |
| | | | hygroscopicus, which encodes the PAT | |
| | | | enzyme. | |
| A-24 | A, B | Agritope Inc. | Reduced accumulation of S- | *Cucumis* |
| | | | adenosylmethionine (SAM), and | *melo* (Melon) |
| | | | consequently reduced ethylene | |
| | | | synthesis, by introduction of the gene | |
| | | | encoding S-adenosylmethionine | |
| | | | hydrolase. | |
| A-25 | CZW-3 | Asgrow | Cucumber mosiac virus (CMV), | *Cucurbita* |
| | | (USA); | zucchini yellows mosaic (ZYMV) and | *pepo* (Squash) |
| | | Seminis | watermelon mosaic virus (WMV) 2 | |
| | | Vegetable Inc. | resistant squash ( Curcurbita pepo) | |
| | | (Canada) | produced by inserting the coat protein | |
| | | | (CP) encoding sequences from each of | |
| | | | these plant viruses into the host | |
| | | | genome. | |
| A-26 | ZW20 | Upjohn (USA); | Zucchini yellows mosaic (ZYMV) and | *Cucurbita* |
| | | Seminis | watermelon mosaic virus (WMV) 2 | *pepo* (Squash) |
| | | Vegetable Inc. | resistant squash (Curcurbita pepo) | |
| | | (Canada) | produced by inserting the coat protein | |
| | | | (CP) encoding sequences from each of | |
| | | | these plant potyviruses into the host | |
| | | | genome. | |
| A-27 | 66 | Florigene Pty | Delayed senescence and sulfonylurea | *Dianthus* |
| | | Ltd. | herbicide tolerant carnations produced | *caryophyllus* ( |
| | | | by inserting a truncated copy of the | Carnation) |
| | | | carnation aminocyclopropane cyclase | |
| | | | (ACC) synthase encoding gene in order | |
| | | | to suppress expression of the | |
| | | | endogenous unmodified gene, which is | |
| | | | required for normal ethylene | |
| | | | biosynthesis. Tolerance to sulfonyl urea | |
| | | | herbicides was via the introduction of a | |
| | | | chlorsulfuron tolerant version of the | |
| | | | acetolactate synthase (ALS) encoding | |
| | | | gene from tobacco. | |
| A-28 | 4, 11, 15, | Florigene Pty | Modified colour and sulfonylurea | *Dianthus* |
| | 16 | Ltd. | herbicide tolerant carnations produced | *caryophyllus* ( |
| | | | by inserting two anthocyanin | Carnation) |
| | | | biosynthetic genes whose expression | |
| | | | results in a violet/mauve | |
| | | | colouration.Tolerance to sulfonyl urea | |
| | | | herbicides was via the introduction of a | |
| | | | chlorsulfuron tolerant version of the | |
| | | | acetolactate synthase (ALS) encoding | |
| | | | gene from tobacco. | |
| A-29 | 959A, | Florigene Pty | Introduction of two anthocyanin | *Dianthus* |
| | 988A, | Ltd. | biosynthetic genes to result in a | *caryophyllus* ( |
| | 1226A, | | violet/mauve colouration; Introduction | Carnation) |
| | 1351A, | | of a variant form of acetolactate | |
| | 1363A, | | synthase (ALS). | |
| | 1400A | | | |
| A-30 | A2704-12, | Aventis | Glufosinate ammonium herbicide | *Glycine max* |
| | A2704-21, | CropScience | tolerant soybean produced by inserting | L. (Soybean) |
| | A5547-35 | | a modified phosphinothricin | |
| | | | acetyltransferase (PAT) encoding gene | |
| | | | from the soil bacterium Streptomyces | |
| | | | viridochromogenes. | |
| A-31 | A5547-127 | Bayer | Glufosinate ammonium herbicide | *Glycine max* |
| | | CropScience | tolerant soybean produced by inserting | L. (Soybean) |
| | | (Aventis | a modified phosphinothricin | |
| | | CropScience(A | acetyltransferase (PAT) encoding gene | |
| | | grEvo)) | from the soil bacterium Streptomyces | |
| | | | viridochromogenes. | |
| A-32 | DP356043 | Pioneer Hi- | Soybean event with two herbicide | *Glycine max* |
| | | Bred | tolerance genes: glyphosate N- | L. (Soybean) |
| | | International | acetlytransferase, which detoxifies | |
| | | Inc. | glyphosate, and a modified acetolactate | |
| | | | synthase (A | |
| A-33 | G94-1, | DuPont | High oleic acid soybean produced by | *Glycine max* |
| | G94-19, | Canada | inserting a second copy of the fatty acid | L. (Soybean) |
| | G168 | Agricultural | desaturase (GmFad2-1) encoding gene | |
| | | Products | from soybean, which resulted in | |
| | | | "silencing" of the endogenous host | |
| | | | gene. | |
| A-34 | GTS 40-3- | Monsanto | Glyphosate tolerant soybean variety | *Glycine max* |
| | 2 | Company | produced by inserting a modified 5- | L. (Soybean) |
| | | | enolpyruvylshikimate-3-phosphate | |
| | | | synthase (EPSPS) encoding gene from | |
| | | | the soil bacterium Agrobacterium | |
| | | | tumefaciens. | |
| A-35 | GU262 | Bayer | Glufosinate ammonium herbicide | *Glycine max* |
| | | CropScience | tolerant soybean produced by inserting | L. (Soybean) |
| | | (Aventis | a modified phosphinothricin | |
| | | CropScience(A | acetyltransferase (PAT) encoding gene | |
| | | grEvo)) | from the soil bacterium Streptomyces | |
| | | | viridochromogenes. | |
| A-36 | MON8978 | Monsanto | Glyphosate-tolerant soybean produced | *Glycine max* |
| | 8 | Company | by inserting a modified 5- | L. (Soybean) |
| | | | enolpyruvylshikimate-3-phosphate | |
| | | | synthase (EPSPS) encoding aroA | |
| | | | (epsps) gene from Agrobacterium | |
| | | | tumefaciens CP4. | |
| A-37 | OT96-15 | Agriculture & | Low linolenic acid soybean produced | *Glycine max* |
| | | Agri-Food | through traditional cross-breeding to | L. (Soybean) |
| | | Canada | incorporate the novel trait from a | |
| | | | naturally occurring fan1 gene mutant | |
| | | | that was selected for low linolenic acid. | |
| A-38 | W62, W98 | Bayer | Glufosinate ammonium herbicide | *Glycine max* |
| | | CropScience | tolerant soybean produced by inserting | L. (Soybean) |
| | | (Aventis | a modified phosphinothricin | |
| | | CropScience(A | acetyltransferase (PAT) encoding gene | |
| | | grEvo)) | from the soil bacterium Streptomyces | |
| | | | hygroscopicus. | |
| A-39 | 15985 | Monsanto | Insect resistant cotton derived by | *Gossypium* |
| | | Company | transformation of the DP50B parent | *hirsutum* |
| | | | variety, which contained event 531 | L. (Cotton) |
| | | | (expressing Cry1Ac protein), with | |
| | | | purified plasmid DNA containing the | |
| | | | cry2Ab gene from B. thuringiensis | |
| | | | subsp. kurstaki. | |
| A-40 | 19-51A | DuPont | Introduction of a variant form of | *Gossypium* |
| | | Canada | acetolactate synthase (ALS). | *hirsutum* |
| | | Agricultural | | L. (Cotton) |
| | | Products | | |
| A-41 | 281-24-236 | DOW | Insect-resistant cotton produced by | *Gossypium* |
| | | AgroSciences | inserting the cry1F gene from Bacillus | *hirsutum* |
| | | LLC | thuringiensisvar. aizawai. The PAT | L. (Cotton) |
| | | | encoding gene from Streptomyces | |
| | | | viridochromogenes was introduced as a | |
| | | | selectable marker. | |
| A-42 | 3006-210- | DOW | Insect-resistant cotton produced by | *Gossypium* |
| | 23 | AgroSciences | inserting the cry1Ac gene from | *hirsutum* |
| | | LLC | Bacillus thuringiensissubsp. kurstaki. | L. (Cotton) |
| | | | The PAT encoding gene from | |
| | | | Streptomyces viridochromogenes was | |
| | | | introduced as a selectable marker. | |
| A-43 | 31807/318 | Calgene Inc. | Insect-resistant and bromoxynil | *Gossypium* |
| | 08 | | herbicide tolerant cotton produced by | *hirsutum* |
| | | | inserting the cry1Ac gene from | L. (Cotton) |
| | | | Bacillus thuringiensis and a nitrilase | |
| | | | encoding gene from Klebsiella | |
| | | | pneumoniae. | |
| A-44 | BXN | Calgene Inc. | Bromoxynil herbicide tolerant cotton | *Gossypium* |
| | | | produced by inserting a nitrilase | *hirsutum* |
| | | | encoding gene from Klebsiella | L. (Cotton) |
| | | | pneumoniae. | |
| A-45 | COT102 | Syngenta | Insect-resistant cotton produced by | *Gossypium* |
| | | Seeds, Inc. | inserting the vip3A(a) gene from | *hirsutum* |
| | | | Bacillus thuringiensisAB88. The APH4 | L. (Cotton) |
| | | | encoding gene from E. coli was | |
| | | | introduced as a selectable marker. | |
| A-46 | DAS- | DOW | WideStrike™, a stacked insect- | *Gossypium* |
| | 21Ø23-5 x | AgroSciences | resistant cotton derived from | *hirsutum* |
| | DAS- | LLC | conventional cross-breeding of parental | L. (Cotton) |
| | 24236-5 | | lines 3006-210-23 (OECD identifier: | |
| | | | DAS-21Ø23-5) and 281-24-236 | |
| | | | (OECD identifier: DAS-24236-5). | |
| A-47 | DAS- | DOW | Stacked insect-resistant and | *Gossypium* |
| | 21Ø23-5 x | AgroSciences | glyphosate-tolerant cotton derived from | *hirsutum* |
| | DAS- | LLC and | conventional cross-breeding of | L. (Cotton) |
| | 24236-5 x | Pioneer Hi- | WideStrike cotton (OECD identifier: | |
| | MON8891 | Bred | DAS-21Ø23-5 x DAS-24236-5) with | |
| | 3 | International | MON88913, known as RoundupReady | |
| | | Inc. | Flex (OECD identifier: MON-88913- | |
| | | | 8). | |
| A-48 | DAS- | DOW | WideStrike™/Roundup Ready® | *Gossypium* |
| | 21Ø23-5 x | AgroSciences | cotton, a stacked insect-resistant and | *hirsutum* |
| | DAS- | LLC | glyphosate-tolerant cotton derived from | L. (Cotton) |
| | 24236-5 x | | conventional cross-breeding of | |
| | MON- | | WideStrike cotton (OECD identifier: | |
| | 01445-2 | | DAS-21Ø23-5 x DAS-24236-5) with | |
| | | | MON1445 (OECD identifier: MON- | |
| | | | Ø1445-2). | |
| A-49 | LLCotton2 | Bayer | Glufosinate ammonium herbicide | *Gossypium* |
| | 5 | CropScience | tolerant cotton produced by inserting a | *hirsutum* |
| | | (Aventis | modified phosphinothricin | L. (Cotton) |
| | | CropScience(A | acetyltransferase (PAT) encoding gene | |
| | | grEvo)) | from the soil bacterium Streptomyces | |
| | | | hygroscopicus. | |
| A-50 | LLCotton2 | Bayer | Stacked herbicide tolerant and insect | *Gossypium* |
| | 5 x | CropScience | resistant cotton combining tolerance to | *hirsutum* |
| | MON1598 | (Aventis | glufosinate ammonium herbicide from | L. (Cotton) |
| | 5 | CropScience(A | LLCotton25 (OECD identifier: ACS- | |
| | | grEvo)) | GHØØ1-3) with resistance to insects | |
| | | | from MON15985 (OECD identifier: | |
| | | | MON-15985-7) | |
| A-51 | MON1445/ | Monsanto | Glyphosate herbicide tolerant cotton | *Gossypium* |
| | 1698 | Company | produced by inserting a naturally | *hirsutum* |
| | | | glyphosate tolerant form of the enzyme | L. (Cotton) |
| | | | 5-enolpyruvyl shikimate-3-phosphate | |
| | | | synthase (EPSPS) from A. tumefaciens | |
| | | | strain CP4. | |
| A-52 | MON1598 | Monsanto | Stacked insect resistant and glyphosate | *Gossypium* |
| | 5 x | Company | tolerant cotton produced by | *hirsutum* |
| | MON8891 | | conventional cross-breeding of the | L. (Cotton) |
| | 3 | | parental lines MON88913 (OECD | |
| | | | identifier: MON-88913-8) and 15985 | |
| | | | (OECD identifier: MON-15985-7). | |
| | | | Glyphosate tolerance is derived from | |
| | | | MON88913 which contains two genes | |
| | | | encoding the enzyme 5- | |
| | | | enolypyruvylshikimate-3-phosphate | |
| | | | synthase (EPSPS) from the CP4 strain | |
| | | | of Agrobacterium tumefaciens. Insect | |
| | | | resistance is derived MON15985 which | |
| | | | was produced by transformation of the | |
| | | | DP50B parent variety, which contained | |
| | | | event 531 (expressing Cry1Ac protein), | |
| | | | with purified plasmid DNA containing | |
| | | | the cry2Ab gene from B. thuringiensis | |
| | | | subsp. kurstaki. | |
| A-53 | MON- | Monsanto | Stacked insect resistant and herbicide | *Gossypium* |
| | 15985-7 x | Company | tolerant cotton derived from | *hirsutum* |
| | MON- | | conventional cross-breeding of the | L. (Cotton) |
| | Ø1445-2 | | parental lines 15985 (OECD identifier: | |
| | | | MON-15985-7) and MON1445 (OECD | |
| | | | identifier: MON-Ø1445-2). | |
| A-54 | MON531/7 | Monsanto | Insect-resistant cotton produced by | *Gossypium* |
| | 57/1076 | Company | inserting the cry1Ac gene from | *hirsutum* |
| | | | Bacillus thuringiensis subsp. kurstaki | L. (Cotton) |
| | | | HD-73 (B.t.k.). | |
| A-55 | MON8891 | Monsanto | Glyphosate herbicide tolerant cotton | *Gossypium* |
| | 3 | Company | produced by inserting two genes | *hirsutum* |
| | | | encoding the enzyme 5- | L. (Cotton) |
| | | | enolypyruvylshikimate-3-phosphate | |
| | | | synthase (EPSPS) from the CP4 strain | |
| | | | of Agrobacterium tumefaciens. | |
| A-56 | MON- | Monsanto | Stacked insect resistant and herbicide | *Gossypium* |
| | ØØ531-6 x | Company | tolerant cotton derived from | *hirsutum* |
| | MON- | | conventional cross-breeding of the | L. (Cotton) |
| | Ø1445-2 | | parental lines MON531 (OECD | |
| | | | identifier: MON-ØØ531-6) and | |
| | | | MON1445 (OECD identifier: MON- | |
| | | | Ø1445-2). | |
| A-57 | X81359 | BASF Inc. | Tolerance to imidazolinone herbicides | *Helianthus* |
| | | | by selection of a naturally occurring | *annuus* (Sunflo |
| | | | mutant. | wer) |
| A-58 | RH44 | BASF Inc. | Selection for a mutagenized version of | *Lens* |
| | | | the enzyme acetohydroxyacid synthase | *culinaris* (Lenti |
| | | | (AHAS), also known as acetolactate | 1) |
| | | | synthase (ALS) or acetolactate | |
| | | | pyruvate- lyase. | |
| A-59 | FP967 | University of | A variant form of acetolactate synthase | *Linum* |
| | | Saskatchewan, | (ALS) was obtained from a | *usitatissimum* |
| | | Crop Dev. | chlorsulfuron tolerant line of A. | L. (Flax, |
| | | Centre | thaliana and used to transform flax. | Linseed) |
| A-60 | 5345 | Monsanto | Resistance to lepidopteran pests | *Lycopersicon* |
| | | Company | through the introduction of the cry1Ac | *esculentum* (To |
| | | | gene from Bacillus thuringiensis subsp. | mato) |
| | | | Kurstaki. | |
| A-61 | 8338 | Monsanto | Introduction of a gene sequence | *Lycopersicon* |
| | | Company | encoding the enzyme 1-amino- | *esculentum* (To |
| | | | cyclopropane-1-carboxylic acid | mato) |
| | | | deaminase (ACCd) that metabolizes the | |
| | | | precursor of the fruit ripening hormone | |
| | | | ethylene. | |
| A-62 | 1345-4 | DNA Plant | Delayed ripening tomatoes produced | *Lycopersicon* |
| | | Technology | by inserting an additional copy of a | *esculentum* (To |
| | | Corporation | truncated gene encoding 1- | mato) |
| | | | aminocyclopropane-1-carboxyllic acid | |
| | | | (ACC) synthase, which resulted in | |
| | | | downregulation of the endogenous | |
| | | | ACC synthase and reduced ethylene | |
| | | | accumulation. | |
| A-63 | 35 1 N | Agritope Inc. | Introduction of a gene sequence | *Lycopersicon* |
| | | | encoding the enzyme S- | *esculentum* (To |
| | | | adenosylmethionine hydrolase that | mato) |
| | | | metabolizes the precursor of the fruit | |
| | | | ripening hormone ethylene | |
| A-64 | B, Da, F | Zeneca Seeds | Delayed softening tomatoes produced | *Lycopersicon* |
| | | | by inserting a truncated version of the | *esculentum* (To |
| | | | polygalacturonase (PG) encoding gene | mato) |
| | | | in the sense or anti-sense orientation in | |
| | | | order to reduce expression of the | |
| | | | endogenous PG gene, and thus reduce | |
| | | | pectin degradation. | |
| A-65 | FLAVR | Calgene Inc. | Delayed softening tomatoes produced | *Lycopersicon* |
| | SAVR | | by inserting an additional copy of the | *esculentum* (To |
| | | | polygalacturonase (PG) encoding gene | mato) |
| | | | in the anti-sense orientation in order to | |
| | | | reduce expression of the endogenous | |
| | | | PG gene and thus reduce pectin | |
| | | | degradation. | |
| A-66 | J101, J163 | Monsanto | Glyphosate herbicide tolerant alfalfa | *Medicago* |
| | | Company and | (lucerne) produced by inserting a gene | *sativa* (Alfalfa) |
| | | Forage | encoding the enzyme 5- | |
| | | Genetics | enolypyruvylshikimate-3-phosphate | |
| | | International | synthase (EPSPS) from the CP4 strain | |
| | | | of Agrobacterium tumefaciens. | |
| A-67 | C/F/93/08- | Societe | Tolerance to the herbicides bromoxynil | *Nicotiana* |
| | 02 | National | and ioxynil by incorporation of the | *tabacum* |
| | | d'Exploitation | nitrilase gene from Klebsiella | L. (Tobacco) |
| | | des Tabacs et | pneumoniae. | |
| | | Allumettes | | |
| A-68 | Vector 21- | Vector | Reduced nicotine content through | *Nicotiana* |
| | 41 | Tobacco Inc. | introduction of a second copy of the | *tabacum* |
| | | | tobacco quinolinic acid | L. (Tobacco) |
| | | | phosphoribosyltransferase (QTPase) in | |
| | | | the antisense orientation. The NPTII | |
| | | | encoding gene from E. coli was | |
| | | | introduced as a selectable marker to | |
| | | | identify transformants. | |
| A-69 | CL121, | BASF Inc. | Tolerance to the imidazolinone | *Oryza* |
| | CL141, | | herbicide, imazethapyr, induced by | *sativa* (Rice) |
| | CFX51 | | chemical mutagenesis of the | |
| | | | acetolactate synthase (ALS) enzyme | |
| | | | using ethyl methanesulfonate (EMS). | |
| A-70 | IMINTA- | BASF Inc. | Tolerance to imidazolinone herbicides | *Oryza* |
| | 1, | | induced by chemical mutagenesis of | *sativa* (Rice) |
| | IMINTA-4 | | the acetolactate synthase (ALS) | |
| | | | enzyme using sodium azide. | |
| A-71 | LLRICE06 | Aventis | Glufosinate ammonium herbicide | *Oryza* |
| | , | CropScience | tolerant rice produced by inserting a | *sativa* (Rice) |
| | LLRICE62 | | modified phosphinothricin | |
| | | | acetyltransferase (PAT) encoding gene | |
| | | | from the soil bacterium Streptomyces | |
| | | | hygroscopicus). | |
| A-72 | LLRICE60 | Bayer | Glufosinate ammonium herbicide | *Oryza* |
| | 1 | CropScience | tolerant rice produced by inserting a | *sativa* (Rice) |
| | | (Aventis | modified phosphinothricin | |
| | | CropScience(A | acetyltransferase (PAT) encoding gene | |
| | | grEvo)) | from the soil bacterium Streptomyces | |
| | | | hygroscopicus). | |
| A-73 | C5 | United States | Plum pox virus (PPV) resistant plum | *Prunus* |
| | | Department of | tree produced through *Agrobacterium-* | *domestica* |
| | | Agriculture - | mediated transformation with a coat | *(Plum)* |
| | | Agricultural | protein (CP) gene from the virus. | |
| | | Research | | |
| | | Service | | |
| A-74 | PWC16 | BASF Inc. | Tolerance to the imidazolinone | *Oryza* |
| | | | herbicide, imazethapyr, induced by | *sativa* (Rice) |
| | | | chemical mutagenesis of the | |
| | | | acetolactate synthase (ALS) enzyme | |
| | | | using ethyl methanesulfonate (EMS). | |
| A-75 | ATBT04- | Monsanto | Colorado potato beetle resistant | *Solanum* |
| | 6, | Company | potatoes produced by inserting the | *tuberosum* |
| | ATBT04- | | cry3A gene from Bacillus thuringiensis | L. (Potato) |
| | 27, | | (subsp. Tenebrionis). | |
| | ATBT04- | | | |
| | 30, | | | |
| | ATBT04- | | | |
| | 31, | | | |
| | ATBT04- | | | |
| | 36, | | | |
| | SPBT02-5, | | | |
| | SPBT02-7 | | | |
| A-76 | BT6, | Monsanto | Colorado potato beetle resistant | *Solanum* |
| | BT10, | Company | potatoes produced by inserting the | *tuberosum* |
| | BT12, | | cry3A gene from Bacillus thuringiensis | L. (Potato) |
| | BT16, | | | |
| | BT17, | | (subsp. Tenebrionis). | |
| | BT18, | | | |
| | BT23 | | | |
| A-77 | RBMT15- | Monsanto | Colorado potato beetle and potato virus | *Solanum* |
| | 101, | Company | Y (PVY) resistant potatoes produced | *tuberosum* |
| | SEMT15- | | by inserting the cry3A gene from | L. (Potato) |
| | 02, | | Bacillus thuringiensis (subsp. | |
| | SEMT15- | | Tenebrionis) and the coat protein | |
| | 15 | | encoding gene from PVY. | |
| A-78 | RBMT21- | Monsanto | Colorado potato beetle and potato | *Solanum* |
| | 129, | Company | leafroll virus (PLRV) resistant potatoes | *tuberosum* |
| | RBMT21- | | produced by inserting the cry3A gene | L. (Potato) |
| | 350, | | from Bacillus thuringiensis (subsp. | |
| | RBMT22- | | Tenebrionis) and the replicase | |
| | 082 | | encoding gene from PLRV. | |
| A-79 | AP205CL | BASF Inc. | Selection for a mutagenized version of | *Triticum* |
| | | | the enzyme acetohydroxyacid synthase | *aestivum* (Whe |
| | | | (AHAS), also known as acetolactate | at) |
| | | | synthase (ALS) or acetolactate | |
| | | | pyruvate- lyase. | |
| A-80 | AP602CL | BASF Inc. | Selection for a mutagenized version of | *Triticum* |
| | | | the enzyme acetohydroxyacid synthase | *aestivum* (Whe |
| | | | (AHAS), also known as acetolactate | at) |
| | | | synthase (ALS) or acetolactate | |
| | | | pyruvate- lyase. | |
| A-81 | BW255-2, | BASF Inc. | Selection for a mutagenized version of | *Triticum* |
| | BW238-3 | | the enzyme acetohydroxyacid synthase | *aestivum* (Whe |
| | | | (AHAS), also known as acetolactate | at) |
| | | | synthase (ALS) or acetolactate | |
| | | | pyruvate- lyase. | |
| A-82 | BW7 | BASF Inc. | Tolerance to imidazolinone herbicides | *Triticum* |
| | | | induced by chemical mutagenesis of | *aestivum* (Whe |
| | | | the acetohydroxyacid synthase (AHAS) | at) |
| | | | gene using sodium azide. | |
| A-83 | MON7180 | Monsanto | Glyphosate tolerant wheat variety | *Triticum* |
| | 0 | Company | produced by inserting a modified 5- | *aestivum* (Whe |
| | | | enolpyruvylshikimate-3-phosphate | at) |
| | | | synthase (EPSPS) encoding gene from | |
| | | | the soil bacterium Agrobacterium | |
| | | | tumefaciens, strain CP4. | |
| A-84 | SWP96500 | Cyanamid | Selection for a mutagenized version of | *Triticum* |
| | 1 | Crop | the enzyme acetohydroxyacid synthase | *aestivum* (Whe |
| | | Protection | (AHAS), also known as acetolactate | at) |
| | | | synthase (ALS) or acetolactate | |
| | | | pyruvate- lyase. | |
| A-85 | Teal 11A | BASF Inc. | Selection for a mutagenized version of | *Triticum* |
| | | | the enzyme acetohydroxyacid synthase | *aestivum* (Whe |
| | | | (AHAS), also known as acetolactate | at) |
| | | | synthase (ALS) or acetolactate | |
| | | | pyruvate- lyase. | |
| A-86 | 176 | Syngenta | Insect-resistant maize produced by | *Zea mays* |
| | | Seeds, Inc. | inserting the cry1Ab gene from | L. (Maize) |
| | | | Bacillus thuringiensis subsp. kurstaki. | |
| | | | The genetic modification affords | |
| | | | resistance to attack by the European | |
| | | | corn borer (ECB). | |
| A-87 | 3751IR | Pioneer Hi- | Selection of somaclonal variants by | *Zea mays* |
| | | Bred | culture of embryos on imidazolinone | L. (Maize) |
| | | International | containing media. | |
| | | Inc. | | |
| A-88 | 676,678, | Pioneer Hi- | Male-sterile and glufosinate | *Zea mays* |
| | 680 | Bred | ammonium herbicide tolerant maize | L. (Maize) |
| | | International | produced by inserting genes encoding | |
| | | Inc. | DNA adenine methylase and | |
| | | | phosphinothricin acetyltransferase | |
| | | | (PAT) from Escherichia coli and | |
| | | | Streptomyces viridochromogenes, | |
| | | | respectively. | |
| A-89 | ACS- | Bayer | Stacked insect resistant and herbicide | *Zea mays* |
| | ZMØØ3-2 | CropScience | tolerant corn hybrid derived from | L. (Maize) |
| | x MON- | (Aventis | conventional cross-breeding of the | |
| | ØØ81Ø-6 | CropScience(A | parental lines T25 (OECD identifier: | |
| | | grEvo)) | ACS-ZMØØ3-2) and MON810 (OECD | |
| | | | identifier:MON-ØØ81Ø-6). | |
| A-90 | B16 | Dekalb | Glufosinate ammonium herbicide | *Zea mays* |
| | (DLL25) | Genetics | tolerant maize produced by inserting | L. (Maize) |
| | | Corporation | the gene encoding phosphinothricin | |
| | | | acetyltransferase (PAT) from | |
| | | | Streptomyces hygroscopicus. | |
| A-91 | BT11 | Syngenta | Insect-resistant and herbicide tolerant | *Zea mays* |
| | (X4334CB | Seeds, Inc. | maize produced by inserting the | L. (Maize) |
| | R, | | cry1Ab gene from Bacillus | |
| | X4734CB | | thuringiensis subsp. kurstaki, and the | |
| | R) | | phosphinothricin N-acetyltransferase | |
| | | | (PAT) encoding gene from S. | |
| | | | viridochromogenes. | |
| A-92 | BT11 x | Syngenta | Stacked insect resistant and herbicide | *Zea mays* |
| | MIR604 | Seeds, Inc. | tolerant maize produced by | L. (Maize) |
| | | | conventional cross breeding of parental | |
| | | | lines BT11 (OECD unique identifier: | |
| | | | SYN-BTØ11-1) and MIR604 (OECD | |
| | | | unique identifier: SYN-IR6Ø5-5). | |
| | | | Resistance to the European Corn Borer | |
| | | | and tolerance to the herbicide | |
| | | | glufosinate ammonium (Liberty) is | |
| | | | derived from BT11, which contains the | |
| | | | cry1Ab gene from Bacillus | |
| | | | thuringiensis subsp. kurstaki, and the | |
| | | | phosphinothricin N-acetyltransferase | |
| | | | (PAT) encoding gene from S. | |
| | | | viridochromogenes. Corn rootworm- | |
| | | | resistance is derived from MIR604 | |
| | | | which contains the mcry3A gene from | |
| | | | Bacillus thuringiensis. | |
| A-93 | BT11 x | Syngenta | Stacked insect resistant and herbicide | *Zea mays* |
| | MIR604 x | Seeds, Inc. | tolerant maize produced by | L. (Maize) |
| | GA21 | | conventional cross breeding of parental | |
| | | | lines BT11 (OECD unique identifier: | |
| | | | SYN-BTØ11-1), MIR604 (OECD | |
| | | | unique identifier: SYN-IR6Ø5-5) and | |
| | | | GA21 (OECD unique identifier: MON- | |
| | | | 00021-9). Resistance to the European | |
| | | | Corn Borer and tolerance to the | |
| | | | herbicide glufosinate ammonium | |
| | | | (Liberty) is derived from BT11, which | |
| | | | contains the cry1Ab gene from Bacillus | |
| | | | thuringiensis subsp. kurstaki, and the | |
| | | | phosphinothricin N-acetyltransferase | |
| | | | (PAT) encoding gene from S. | |
| | | | viridochromogenes. Corn rootworm- | |
| | | | resistance is derived from MIR604 | |
| | | | which contains the mcry3A gene from | |
| | | | Bacillus thuringiensis. Tolerance to | |
| | | | glyphosate herbcicide is derived from | |
| | | | GA21 which contains a a modified | |
| | | | EPSPS gene from maize. | |
| A-94 | CBH-351 | Aventis | Insect-resistant and glufosinate | *Zea mays* |
| | | CropScience | ammonium herbicide tolerant maize | L. (Maize) |
| | | | developed by inserting genes encoding | |
| | | | Cry9C protein from Bacillus | |
| | | | thuringiensis subsp tolworthi and | |
| | | | phosphinothricin acetyltransferase | |
| | | | (PAT) from Streptomyces | |
| | | | hygroscopicus. | |
| A-95 | DAS- | DOW | Lepidopteran insect resistant and | *Zea mays* |
| | 06275-8 | AgroSciences | glufosinate ammonium herbicide- | L. (Maize) |
| | | LLC | tolerant maize variety produced by | |
| | | | inserting the cry1F gene from Bacillus | |
| | | | thuringiensis var aizawai and the | |
| | | | phosphinothricin acetyltransferase | |
| | | | (PAT) from Streptomyces | |
| | | | hygroscopicus. | |
| A-96 | DAS- | DOW | Corn rootworm-resistant maize | *Zea mays* |
| | 59122-7 | AgroSciences | produced by inserting the cry34Ab1 | L. (Maize) |
| | | LLC and | and cry35Ab1 genes from Bacillus | |
| | | Pioneer Hi- | thuringiensis strain PS149B1. The PAT | |
| | | Bred | encoding gene from Streptomyces | |
| | | International | viridochromogenes was introduced as a | |
| | | Inc. | selectable marker. | |
| A-97 | DAS- | DOW | Stacked insect resistant and herbicide | *Zea mays* |
| | 59122-7 x | AgroSciences | tolerant maize produced by | L. (Maize) |
| | NK603 | LLC and | conventional cross breeding of parental | |
| | | Pioneer Hi- | lines DAS-59122-7 (OECD unique | |
| | | Bred | identifier: DAS-59122-7) with NK603 | |
| | | International | (OECD unique identifier: MON- | |
| | | Inc. | ØØ6Ø3-6). Corn rootworm-resistance | |
| | | | is derived from DAS-59122-7 which | |
| | | | contains the cry34Ab1 and cry35Ab1 | |
| | | | genes from Bacillus thuringiensis strain | |
| | | | PS149B1. Tolerance to glyphosate | |
| | | | herbcicide is derived from NK603. | |
| A-98 | DAS- | DOW | Stacked insect resistant and herbicide | *Zea mays* |
| | 59122-7 x | AgroSciences | tolerant maize produced by | L. (Maize) |
| | TC1507 x | LLC and | conventional cross breeding of parental | |
| | NK603 | Pioneer Hi- | lines DAS-59122-7 (OECD unique | |
| | | Bred | identifier: DAS-59122-7) and TC1507 | |
| | | International | (OECD unique identifier: DAS- | |
| | | Inc. | Ø15Ø7-1) with NK603 (OECD unique | |
| | | | identifier: MON-ØØ6Ø3-6). Corn | |
| | | | rootworm-resistance is derived from | |
| | | | DAS-59122-7 which contains the | |
| | | | cry34Ab1 and cry35Ab1 genes from | |
| | | | Bacillus thuringiensis strain PS149B1. | |
| | | | Lepidopteran resistance and toleraance | |
| | | | to glufosinate ammonium herbicide is | |
| | | | derived from TC1507. Tolerance to | |
| | | | glyphosate herbcicide is derived from | |
| | | | NK603. | |
| A-99 | DAS- | DOW | Stacked insect resistant and herbicide | *Zea mays* |
| | Ø15Ø7-1 x | AgroSciences | tolerant corn hybrid derived from | L. (Maize) |
| | MON- | LLC | conventional cross-breeding of the | |
| | ØØ6Ø3-6 | | parental lines 1507 (OECD identifier: | |
| | | | DAS-Ø15Ø7-1) and NK603 (OECD | |
| | | | identifier: MON-ØØ6Ø3-6). | |
| A-100 | DBT418 | Dekalb | Insect-resistant and glufosinate | *Zea mays* |
| | | Genetics | ammonium herbicide tolerant maize | L. (Maize) |
| | | Corporation | developed by inserting genes encoding | |
| | | | Cry1AC protein from Bacillus | |
| | | | thuringiensis subsp kurstaki and | |
| | | | phosphinothricin acetyltransferase | |
| | | | (PAT) from Streptomyces | |
| | | | hygroscopicus | |
| A-101 | DK404SR | BASF Inc. | Somaclonal variants with a modified | *Zea mays* |
| | | | acetyl-CoA-carboxylase (ACCase) | L. (Maize) |
| | | | were selected by culture of embryos on | |
| | | | sethoxydim enriched medium. | |
| A-102 | Event 3272 | Syngenta | Maize line expressing a heat stable | *Zea mays* |
| | | Seeds, Inc. | alpha-amylase gene amy797E for use | L. (Maize) |
| | | | in the dry-grind ethanol process. The | |
| | | | phosphomannose isomerase gene from | |
| | | | E.coli was used as a selectable marker. | |
| A-103 | EXP1910I | Syngenta | Tolerance to the imidazolinone | *Zea mays* |
| | T | Seeds, Inc. | herbicide, imazethapyr, induced by | L. (Maize) |
| | | (formerly | chemical mutagenesis of the | |
| | | Zeneca Seeds) | acetolactate synthase (ALS) enzyme | |
| | | | using ethyl methanesulfonate (EMS). | |
| A-104 | GA21 | Monsanto | Introduction, by particle bombardment, | *Zea mays* |
| | | Company | of a modified 5-enolpyruvyl shikimate- | L. (Maize) |
| | | | 3-phosphate synthase (EPSPS), an | |
| | | | enzyme involved in the shikimate | |
| | | | biochemical pathway for the | |
| | | | production of the aromatic amino acids. | |
| A-105 | IT | Pioneer Hi- | Tolerance to the imidazolinone | *Zea mays* |
| | | Bred | herbicide, imazethapyr, was obtained | L. (Maize) |
| | | International | by in vitro selection of somaclonal | |
| | | Inc. | variants. | |
| A-106 | LY038 | Monsanto | Altered amino acid composition, | *Zea mays* |
| | | Company | specifically elevated levels of lysine, | L. (Maize) |
| | | | through the introduction of the cordapA | |
| | | | gene, derived from Corynebacterium | |
| | | | glutamicum, encoding the enzyme | |
| | | | dihydrodipicolinate synthase | |
| | | | (cDHDPS). | |
| A-107 | MIR604 | Syngenta | Corn rootworm resistant maize | *Zea mays* |
| | | Seeds, Inc. | produced by transformation with a | L. (Maize) |
| | | | modified cry3A gene. The | |
| | | | phosphomannose isomerase gene from E.coli was used as a selectable marker. | |
| A-108 | MIR604 x | Syngenta | Stacked insect resistant and herbicide | *Zea mays* |
| | GA21 | Seeds, Inc. | tolerant maize produced by | L. (Maize) |
| | | | conventional cross breeding of parental | |
| | | | lines MIR604 (OECD unique | |
| | | | identifier: SYN-IR6Ø5-5) and GA21 | |
| | | | (OECD unique identifier: MON- | |
| | | | 00021-9). Corn rootworm-resistance | |
| | | | is derived from MIR604 which | |
| | | | contains the mcry3A gene from | |
| | | | Bacillus thuringiensis. Tolerance to | |
| | | | glyphosate herbcicide is derived from | |
| | | | GA21. | |
| A-109 | MON8010 | Monsanto | Insect-resistant maize produced by | *Zea mays* |
| | 0 | Company | inserting the cry1Ab gene from | L. (Maize) |
| | | | Bacillus thuringiensis subsp. kurstaki. | |
| | | | The genetic modification affords | |
| | | | resistance to attack by the European | |
| | | | corn borer (ECB). | |
| A-110 | MON802 | Monsanto | Insect-resistant and glyphosate | *Zea mays* |
| | | Company | herbicide tolerant maize produced by | L. (Maize) |
| | | | inserting the genes encoding the | |
| | | | Cry1Ab protein from Bacillus | |
| | | | thuringiensis and the 5- | |
| | | | enolpyruvylshikimate-3-phosphate | |
| | | | synthase (EPSPS) from A. tumefaciens | |
| | | | strain CP4. | |
| A-111 | MON809 | Pioneer Hi- | Resistance to European corn borer | *Zea mays* |
| | | Bred | (Ostrinia nubilalis) by introduction of a | L. (Maize) |
| | | International | synthetic cry1Ab gene. Glyphosate | |
| | | Inc. | resistance via introduction of the | |
| | | | bacterial version of a plant enzyme, 5- | |
| | | | enolpyruvyl shikimate-3-phosphate | |
| | | | synthase (EPSPS). | |
| A-112 | MON810 | Monsanto | Insect-resistant maize produced by | *Zea mays* |
| | | Company | inserting a truncated form of the | L. (Maize) |
| | | | cry1Ab gene from Bacillus | |
| | | | thuringiensis subsp. kurstaki HD-1. | |
| | | | The genetic modification affords | |
| | | | resistance to attack by the European | |
| | | | corn borer (ECB). | |
| A-113 | MON810 x | Monsanto | Stacked insect resistant and glyphosate | *Zea mays* |
| | MON8801 | Company | tolerant maize derived from | L. (Maize) |
| | 7 | | conventional cross-breeding of the | |
| | | | parental lines MON810 (OECD | |
| | | | identifier: MON-ØØ81Ø-6) and | |
| | | | MON88017 (OECD identifier:MON- | |
| | | | 88Ø17-3). European corn borer (ECB) | |
| | | | resistance is derived from a truncated | |
| | | | form of the cry1Ab gene from Bacillus | |
| | | | thuringiensis subsp. kurstaki HD-1 | |
| | | | present in MON810. Corn rootworm | |
| | | | resistance is derived from the cry3Bb1 | |
| | | | gene from Bacillus thuringiensis | |
| | | | subspecies kumamotoensis strain | |
| | | | EG4691 present in MON88017. | |
| | | | Glyphosate tolerance is derived from a | |
| | | | 5-enolpyruvylshikimate-3-phosphate | |
| | | | synthase (EPSPS) encoding gene from | |
| | | | Agrobacterium tumefaciens strain CP4 | |
| | | | present in MON88017. | |
| A-114 | MON832 | Monsanto | Introduction, by particle bombardment, | *Zea mays* |
| | | Company | of glyphosate oxidase (GOX) and a | L. (Maize) |
| | | | modified 5-enolpyruvyl shikimate-3- | |
| | | | phosphate synthase (EPSPS), an | |
| | | | enzyme involved in the shikimate | |
| | | | biochemical pathway for the | |
| | | | production of the aromatic amino acids. | |
| A-115 | MON863 | Monsanto | Corn root worm resistant maize | *Zea mays* |
| | | Company | produced by inserting the cry3Bb1 | L. (Maize) |
| | | | gene from Bacillus thuringiensis subsp. | |
| | | | kumamotoensis. | |
| A-116 | MON8801 | Monsanto | Corn rootworm-resistant maize | *Zea mays* |
| | 7 | Company | produced by inserting the cry3Bb1 | L. (Maize) |
| | | | gene from Bacillus thuringiensis | |
| | | | subspecies kumamotoensis strain | |
| | | | EG4691. Glyphosate tolerance derived | |
| | | | by inserting a 5-enolpyruvylshikimate- | |
| | | | 3-phosphate synthase (EPSPS) | |
| | | | encoding gene from Agrobacterium | |
| | | | tumefaciens strain CP4. | |
| A-117 | MON8903 | Monsanto | Maize event expressing two different | *Zea mays* |
| | 4 | Company | insecticidal proteins from Bacillus | L. (Maize) |
| | | | thuringiensis providing resistance to | |
| | | | number of lepidopteran pests. | |
| A-118 | MON8903 | Monsanto | Stacked insect resistant and glyphosate | *Zea mays* |
| | 4 x | Company | tolerant maize derived from | L. (Maize) |
| | MON8801 | | conventional cross-breeding of the | |
| | 7 | | parental lines MON89034 (OECD | |
| | | | identifier: MON-89Ø34-3) and | |
| | | | MON88017 (OECD identifier:MON- | |
| | | | 88017-3). Resistance to Lepiopteran | |
| | | | insects is derived from two crygenes | |
| | | | present in MON89043. Corn rootworm | |
| | | | resistance is derived from a single cry | |
| | | | genes and glyphosate tolerance is | |
| | | | derived from the 5- | |
| | | | enolpyruvylshikimate-3-phosphate | |
| | | | synthase (EPSPS) encoding gene from | |
| | | | Agrobacterium tumefaciens present in | |
| | | | MON88017. | |
| A-119 | MON- | Monsanto | Stacked insect resistant and herbicide | *Zea mays* |
| | ØØ6Ø3-6 | Company | tolerant corn hybrid derived from | L. (Maize) |
| | x MON- | | conventional cross-breeding of the | |
| | ØØ81Ø-6 | | parental lines NK603 (OECD | |
| | | | identifier: MON-ØØ6Ø3-6) and | |
| | | | MON810 (OECD identifier: MON- | |
| | | | ØØ81Ø-6). | |
| A-120 | MON- | Monsanto | Stacked insect resistant and enhanced | *Zea mays* |
| | ØØ81Ø-6 | Company | lysine content maize derived from | L. (Maize) |
| | x LY038 | | conventional cross-breeding of the | |
| | | | parental lines MON810 (OECD | |
| | | | identifier: MON-ØØ81Ø-6) and LY038 | |
| | | | (OECD identifier: REN-ØØØ38-3). | |
| A-121 | MON- | Monsanto | Stacked insect resistant and herbicide | *Zea mays* |
| | 00863-5 x | Company | tolerant corn hybrid derived from | L. (Maize) |
| | MON- | | conventional cross-breeding of the | |
| | 00603-6 | | parental lines MON863 (OECD | |
| | | | identifier:MON-ØØ863-5) and NK603 | |
| | | | (OECD identifier: MON-ØØ6Ø3-6). | |
| A-122 | MON- | Monsanto | Stacked insect resistant corn hybrid | *Zea mays* |
| | 00863-5 x | Company | derived from conventional cross- | L. (Maize) |
| | MON- | | breeding of the parental lines MON863 | |
| | ØØ81Ø-6 | | (OECD identifier: MON-ØØ863-5) and | |
| | | | MON810 (OECD identifier: MON- | |
| | | | ØØ81Ø-6) | |
| A-123 | MON- | Monsanto | Stacked insect resistant and herbicide | *Zea mays* |
| | 00863-5 x | Company | tolerant corn hybrid derived from | L. (Maize) |
| | MON- | | conventional cross-breeding of the | |
| | ØØ81Ø-6 | | stacked hybrid MON-ØØ863-5 x | |
| | x MON- | | MON-ØØ81Ø-6 and NK603 (OECD | |
| | 00603-6 | | identifier:MON-ØØ6Ø3-6). | |
| A-124 | MON- | Monsanto | Stacked insect resistant and herbicide | *Zea mays* |
| | ØØØ21-9 | Company | tolerant corn hybrid derived from | L. (Maize) |
| | x MON- | | conventional cross-breeding of the | |
| | ØØ81Ø-6 | | parental lines GA21 (OECD | |
| | | | identifider: MON-ØØØ21-9) and | |
| | | | MON810 (OECD identifier: MON- | |
| | | | ØØ81Ø-6). | |
| A-125 | MS3 | Bayer | Male sterility caused by expression of | *Zea mays* |
| | | CropScience | the barnase ribonuclease gene from | L. (Maize) |
| | | (Aventis | Bacillus amyloliquefaciens; PPT | |
| | | CropScience(A | resistance was via PPT- | |
| | | grEvo)) | acetyltransferase (PAT). | |
| A-126 | MS6 | Bayer | Male sterility caused by expression of | *Zea mays* |
| | | CropScience | the barnase ribonuclease gene from | L. (Maize) |
| | | (Aventis | Bacillus amyloliquefaciens; PPT | |
| | | CropScience(A | resistance was via PPT- | |
| | | grEvo)) | acetyltransferase (PAT). | |
| A-127 | NK603 | Monsanto | Introduction, by particle bombardment, | *Zea mays* |
| | | Company | of a modified 5-enolpyruvyl shikimate- | L. (Maize) |
| | | | 3-phosphate synthase (EPSPS), an | |
| | | | enzyme involved in the shikimate | |
| | | | biochemical pathway for the | |
| | | | production of the aromatic amino acids. | |
| A-128 | SYN- | Syngenta | Stacked insect resistant and herbicide | *Zea mays* |
| | BTØ11-1 x | Seeds, Inc. | tolerant maize produced by | L. (Maize) |
| | MON- | | conventional cross breeding of parental | |
| | ØØØ21-9 | | lines BT11 (OECD unique identifier: | |
| | | | SYN-BTØ11-1) and GA21 (OECD | |
| | | | unique identifier: MON-ØØØ21-9). | |
| A-129 | T14, T25 | Bayer | Glufosinate herbicide tolerant maize | *Zea mays* |
| | | CropScience | produced by inserting the | L. (Maize) |
| | | (Aventis | phosphinothricin N-acetyltransferase | |
| | | CropScience(A | (PAT) encoding gene from the aerobic | |
| | | grEvo)) | actinomycete Streptomyces | |
| | | | viridochromogenes. | |
| A-130 | TC1507 | Mycogen (c/o | Insect-resistant and glufosinate | *Zea mays* |
| | | Dow | ammonium herbicide tolerant maize | L. (Maize) |
| | | AgroSciences); | produced by inserting the cry1F gene | |
| | | Pioneer (c/o | from Bacillus thuringiensis var. aizawai | |
| | | Dupont) | and the phosphinothricin N- | |
| | | | acetyltransferase encoding gene from | |
| | | | Streptomyces viridochromogenes. | |
| A-131 | TC1507 x | DOW | Stacked insect resistant and herbicide | *Zea mays* |
| | DAS- | AgroSciences | tolerant maize produced by | L. (Maize) |
| | 59122-7 | LLC and | conventional cross breeding of parental | |
| | | Pioneer Hi- | lines TC1507 (OECD unique identifier: | |
| | | Bred | DAS-Ø15Ø7-1) with DAS-59122-7 | |
| | | International | (OECD unique identifier: DAS-59122- | |
| | | Inc. | 7). Resistance to lepidopteran insects is | |
| | | | derived from TC1507 due the presence | |
| | | | of the cry1F gene from Bacillus | |
| | | | thuringiensis var. aizawai. Corn | |
| | | | rootworm-resistance is derived from | |
| | | | DAS-59122-7 which contains the | |
| | | | cry34Ab1 and cry35Ab1 genes from | |
| | | | Bacillus thuringiensis strain PS149B1. | |
| | | | Tolerance to glufosinate ammonium | |
| | | | herbcicide is derived from TC1507 | |
| | | | from the phosphinothricin N- | |
| | | | acetyltransferase encoding gene from | |
| | | | Streptomyces viridochromogenes. | |
| | | | | |

In one embodiment of the invention, the plants A-1 to A-131 of Table A, in total, or parts thereof, or propagation material of said plant are treated or contacted with Fluopyram alone, or in the form of compositions comprising Fluopyram.

### Table B

Non-exhaustive list of transgenic plants to work the invention from on APHIS database of the United States Department of Agriculture (USDA). The database can be found on : http://www.aphis.usda.gov/animal_welfare/efoia/index.shtml

Abbreviations used in this table:
CMV-cucumber mosaic virus
CPB-colorado potato beetle
PLRV- potato leafroll virus
PRSV-papaya ringspot virus
PVY-potato virus Y
WMV2- watermelon mosaic virus 2
ZYMV-zucchini yellow mosaic virus

| **No.** | **Petition** | **Extension of Petition Number** | **Institution** | **Plant** | **Trait** | **Transformation Event or Line** | **Final EA & Determin ation** |
|---|---|---|---|---|---|---|---|
| B-1 | 08-315-01p | | Florigene | Rose | Altered Flower Color | Rosa X hybrida | |
| B-2 | 07-253-01p | | Syngenta | Corn | Lepidopteran resistant | MIR-162 Maize | |
| B-3 | 07-152-01p | | Pioneer | Corn | glyphosate & Imidazolinone tolerant | HT-98140 | |
| B-4 | 07-108-01p | | Syngenta | Cotton | Lepidopteran Resistant | COT67B | |
| B-5 | 06-354- 01p | | Pioneer | Soybean | High Oleic Acid | DP-3Ø5423-1 | |
| B-6 | 06-332- 01p | | Bayer CropScience | Cotton | Glyphosate tolerant | GHB614 | |
| B-7 | 05-280-01p | | Syngenta | Corn | Thermostable alpha-amylase | 3272 | |
| B-8 | 04-337-01p | | University of Florida | Papaya | Papaya Ringspot Virus Resistant | X17-2 | |
| B-9 | 04-110-01p | | Monsanto & Forage Genetics | Alfalfa | Glyphosate Tolerant | J101, J163 | 04-110- 01p com |
| B-10 | 03-104-01p | | Monsanto & Scotts | Creeping bentgrass | Glyphosate Tolerant | ASR368 | |
| B-11 | 06-298- 01p | | Monsanto | Corn | European Corn Borer resistant | MON 89034 | 06-298- 01p com |
| B- 12 | 06-271- 01p | | Pioneer | Soybean | Glyphosate & acetolactate synthase tolerant | 356043 (DP-356Ø43- 5) | 06-271- 01p com |
| B-13 | 06-234- 01p | 98-329- 01p | Bayer CropScience | Rice | Phosphinothricin tolerant | LLRICE601 | 06-234- 01p com |
| B- 14 | 06-178- 01p | | Monsanto | Soybean | Glyphosate tolerant | MON 89788 | 06-178- 01p com |
| B- 15 | 04-362- 01p | | Syngenta | Corn | Corn Rootworm Protected | MIR604 | 04-362- 01p com |
| B- 16 | 04-264- 01p | | ARS | Plum | Plum Pox Virus Resistant | C5 | 04-264- 01p com |
| B- 17 | 04-229- 01p | | Monsanto | Corn | High Lysine | LY038 | 04-229- 01p com |
| B- 18 | 04-125- 01p | | Monsanto | Corn | Corn Rootworm Resistant | MON 88017 | 04-125- 01p com |
| B- 19 | 04-086- 01p | | Monsanto | Cotton | Glyphosate Tolerant | MON 88913 | 04-086- 01p com |
| B- 20 | 03-353- 01p | | Dow | Corn | Corn Rootworm Resistant | 59122 | 03-353- 01p com |
| B- 21 | 03-323- 01p | | Monsanto | Sugar Beet | Glyphosate Tolerant | H7-1 | 03-323- 01p com |
| B- 22 | 03-181- 01p | 00-136- 01p | Dow | Corn | Lepidopteran Resistant & Phosphinothricin tolerant | TC-6275 | 03-181- 01p com |
| B- 23 | 03-155- 01p | | Syngenta | Cotton | Lepidopteran Resistant | COT 102 | 03-155- 01p com |
| B- 24 | 03-036- 01p | | Mycogen/Dow | Cotton | Lepidopteran Resistant | 281-24-236 | 03-036- 01p com |
| B- 25 | 03-036- 02p | | Mycogen/Dow | Cotton | Lepidopteran Resistant | 3006-210-23 | 03-036- 02p com |
| B- 26 | 02-042- 01p | | Aventis | Cotton | Phosphinothericin tolerant | LLCotton25 | 02-042- 01p com |
| B- 27 | 01-324- 01p | 98-216- 01p | Monsanto | Rapeseed | Glyphosate tolerant | RT200 | 01-324- 01p com |
| B- 28 | 01-206- 01p | 98-278- 01p | Aventis | Rapeseed | Phosphinothricin tolerant & pollination control | MS1 & RF1/RF2 | 01-206- 01p com |
| B- 29 | 01-206- 02p | 97-205- 01p | Aventis | Rapeseed | Phosphinothricin tolerant | Topas 19/2 | 01-206- 02p com |
| B- 30 | 01-137- 01p | | Monsanto | Corn | Corn Rootworm Resistant | MON 863 | 01-137- 01p com |
| B- 31 | 01-121- 01p | | Vector | Tobacco | Reduced nicotine | Vector 21-41 | 01-121- 01p com |
| B- 32 | 00-342- 01p | | Monsanto | Cotton | Lepidopteran resistant | Cotton Event 15985 | 00-342- 01p com |
| B- 33 | 00-136- 01p | | Mycogen c/o Dow & Pioneer | Corn | Lepidopteran resistant phosphinothricin tolerant | Line 1507 | 00-136- 01p com |
| B- 34 | 00-011- 01p | 97-099- 01p | Monsanto | Corn | Glyphosate tolerant | NK603 | 00-011- 01p com |
| B- 35 | 99-173- 01p | 97-204- 01p | Monsanto | Potato | PLRV & CPB resistant | RBMT22-82 | 99-173- 01p com |
| B- 36 | 98-349- 01p | 95-228- 01p | AgrEvo | Corn | Phosphinothricin tolerant and Male sterile | MS6 | 98-349- 01p com |
| B- 37 | 98-335- 01p | | U. of Saskatchewan | Flax | Tolerant to soil residues of sulfonyl urea herbicide | CDC Triffid | 98-335- 01p com |
| B- 38 | 98-329- 01p | | AgrEvo | Rice | Phosphinothricin tolerant | LLRICE06, LLRICE62 | 98-329- 01p com |
| B- 39 | 98-278- 01p | | AgrEvo | Rapeseed | Phosphinothricin tolerant & Pollination control | MS8 & RF3 | 98-278- 01p com |
| B- 40 | 98-238- 01p | | AgrEvo | Soybean | Phosphinothricin tolerant | GU262 | 98-238- 01p com |
| B- 41 | 98-216- 01p | | Monsanto | Rapeseed | Glyphosate tolerant | RT73 | 98-216- 01p com |
| B- 42 | 98-173- 01p | | Novartis Seeds & Monsanto | Beet | Glyphosate tolerant | GTSB77 | 98-173- 01p com |
| B- 43 | 98-014- 01p | 96-068- 01p | AgrEvo | Soybean | Phosphinothricin tolerant | A5547-127 | 98-014- 01p com |
| B- 44 | 97-342- 01p | | Pioneer | Corn | Male sterile & Phosphinothricin tolerant | 676,678,680 | 97-342- 01p com |
| B- 45 | 97-339- 01p | | Monsanto | Potato | CPB & PVY resistant | RBMT15-101, SEMT15-02, SEMT15-15 | 97-339- 01p com |
| B- 46 | 97-336- 01p | | AgrEvo | Beet | Phosphinothricin tolerant | T-120-7 | 97-336- 01p com |
| B- 47 | 97-287- 01p | | Monsanto | Tomato | Lepidopteran resistant | 5345 | 97-287- 01p com |
| B- 48 | 97-265- 01p | | AgrEvo | Corn | Phosphinothricin tolerant & Lep. resistant | CBH-351 | 97-265- 01p com |
| B- 49 | 97-205- 01p | | AgrEvo | Rapeseed | Phosphinothricin tolerant | T45 | 97-205- 01p com |
| B- 50 | 97-204- 01p | | Monsanto | Potato | CPB & PLRV resistant | RBMT21-129 & RBMT21- 350 | 97-204- 01p com |
| B- 51 | 97-148- 01p | | Bejo | Cichorium intybus | Male sterile | RM3-3, RM3- 4, RM3-6 | 97-148- 01p com |
| B- 52 | 97-099- 01p | | Monsanto | Corn | Glyphosate tolerant | GA21 | 97-099- 01p com |
| B- 53 | 97-013- 01p | | Calgene | Cotton | Bromoxynil tolerant & Lepidopteran resistant | Events 31807 & 31808 | 97-013- 01p com |
| B- 54 | 97-008- 01p | | Du Pont | Soybean | Oil profile altered | G94-1, G94- 19, G-168 | 97-008- 01p com |
| B- 55 | 96-317- 01p | | Monsanto | Corn | Glyphosate tolerant & ECB resistant | MON802 | 96-317- 01p com |
| B- 56 | 96-291- 01p | | DeKalb | Corn | European Corn Borer resistant | DBT418 | 96-291- 01p com |
| B- 57 | 96-248- 01p | 92-196- 01p | Calgene | Tomato | Fruit ripening altered | 1 additional FLAVRSAVR line | 96-248- 01p com |
| B- 58 | 96-068- 01p | | AgrEvo | Soybean | Phosphinothricin tolerant | W62, W98, A2704- 12, A2704-21, A5547-35 | 96-068- 01p com |
| B- 59 | 96-051- 01p | | Cornell U | Papaya | PRSV resistant | 55-1,63-1 | 96-051- 01p com |
| B- 60 | 96-017- 01p | 95-093- 01p | Monsanto | Corn | European Corn Borer resistant | MON809 & MON810 | 96-017- 01p com |
| B- 61 | 95-352- 01p | | Asgrow | Squash | CMV, ZYMV, WMV2 resistant | CZW-3 | 95-352- 01p com |
| B- 62 | 95-338- 01p | | Monsanto | Potato | CPB resistant | SBT02-5 & - 7, ATBT04-6 &-27, -30, - 31, -36 | 95-338- 01p com |
| B- 63 | 95-324- 01p | | Agritope | Tomato | Fruit ripening altered | 35 1 N | 95-324- 01p com |
| B- 64 | 95-256- 01p | | Du Pont | Cotton | Sulfonylurea tolerant | 19-51a | 95-256- 01p com |
| B- 65 | 95-228- 01p | | Plant Genetic Systems | Corn | Male sterile | MS3 | 95-228- 01p com |
| B- 66 | 95-195-01p | | Northrup King | Corn | European Corn Borer resistant | Bt11 | 95-195-01p com |
| B- 67 | 95-179- 01p | 92-196- 01p | Calgene | Tomato | Fruit ripening altered | 2 additional FLAVRSAVR lines | 95-179- 01p com |
| B- 68 | 95-145- 01p | | DeKalb | Corn | Phosphinothricin tolerant | B16 | 95-145- 01p com |
| B- 69 | 95-093- 01p | | Monsanto | Corn | Lepidopteran resistant | MON 80100 | 95-093- 01p com |
| B- 70 | 95-053- 01p | | Monsanto | Tomato | Fruit ripening altered | 8338 | 95-053- 01p com |
| B- 71 | 95-045- 01p | | Monsanto | Cotton | Glyphosate tolerant | 1445, 1698 | 95-045- 01p com |
| B- 72 | 95-030- 01p | 92-196- 01p | Calgene | Tomato | Fruit ripening altered | 20 additional FLAVRSAVR lines | 95-030- 01p com |
| B- 73 | 94-357- 01p | | AgrEvo | Corn | Phosphinothricin tolerant | T14, T25 | 94-357- 01p com |
| B- 74 | 94-319- 01p | | Ciba Seeds | Corn | Lepidopteran resistant | Event 176 | 94-319- 01p com |
| B- 75 | 94-308- 01p | | Monsanto | Cotton | Lepidopteran resistant | 531,757, 1076 | 94-308- 01p com |
| B- 76 | 94-290- 01p | | Zeneca & Petoseed | Tomato | Fruit polygalacturonase level decreased | B, Da, F | 94-290- 01p com |
| B- 77 | 94-257- 01p | | Monsanto | Potato | Coleopteran resistant | BT6, BT10, BT12, BT16, BT17, BT18, BT23 | 94-257- 01p com |
| B- 78 | 94-230- 01p | 92-196- 01p | Calgene | Tomato | Fruit ripening altered | 9 additional FLAVRSAVR lines | 94-230- 01p com |
| B- 79 | 94-228- 01p | | DNA Plant Tech | Tomato | Fruit ripening altered | 1345-4 | 94-228- 01p com |
| B- 80 | 94-227- 01p | 92-196- 01p | Calgene | Tomato | Fruit ripening altered | Line N73 1436-111 | 94-227- 01p com |
| B- 81 | 94-090- 01p | | Calgene | Rapeseed | Oil profile altered | pCGN3828- 212/86- 18 & 23 | 94-090- 01p com |
| B- 82 | 93-258- 01p | | Monsanto | Soybean | Glyphosate tolerant | 40-3-2 | 93-258- 01p com |
| B- 83 | 93-196- 01p | | Calgene | Cotton | Bromoxynil tolerant | BXN | 93-196- 01p com |
| B- 84 | 92-204- 01p | | Upjohn | Squash | WMV2 & ZYMV resistant | ZW-20 | 92-204- 01p com |
| B- 85 | 92-196- 01p | | Calgene | Tomato | Fruit ripening altered | FLAVR SAVR | 92-196- 01p com |

In one embodiment of the invention, the plants B-1 to B-85 of Table B, in total, or parts thereof, or propagation material of said plant are treated or contacted with Fluopyram alone, or in the form of compositions comprising Fluopyram.

**Table C**

| Non-exhaustive list of traits to work the invention with references to documents in which they are decribed. | | |
|---|---|---|
| **No.** | **Trait** | **Reference** |
| C-1 | Water use efficiency | WO 2000/073475 |
| | Nitrogen use efficiency | WO 1995/009911 |
| | | WO 1997/030163 |
| | | WO 2007/092704 |
| | | WO 2007/076115 |
| | | WO 2005/103270 |
| | | WO 2002/002776 |
| C-2 | Improved photosynthesis | WO 2008/056915 |
| | | WO 2004/101751 |
| C-3 | Nematode resistance | WO 1995/020669 |
| | | WO 2001/051627 |
| | | WO 2008/139334 |
| | | WO 2008/095972 |
| | | WO 2006/085966 |
| | | WO 2003/033651 |
| | | WO 1999/060141 |
| | | WO 1998/012335 |
| | | WO 1996/030517 |
| | | WO 1993/018170 |
| C-4 | Reduced pod dehiscence | WO 2006/009649 |
| | | WO 2004/113542 |
| | | WO 1999/015680 |
| | | WO 1999/000502 |
| | | WO 1997/013865 |
| | | WO 1996/030529 |
| | | WO 1994/023043 |
| C-5 | Aphid resistance | WO 2006/125065 |
| | | WO 1997/046080 |
| | | WO 2008/067043 |
| | | WO 2004/072109 |
| C-6 | Sclerotinia resistance | WO 2006/135717 |
| | | WO 2006/055851 |
| | | WO 2005/090578 |
| | | WO 2005/000007 |
| | | WO 2002/099385 |
| | | WO 2002/061043 |
| C-7 | Botrytis resistance | WO 2006/046861 |
| | | WO 2002/085105 |
| C-8 | Bremia resistance | US 20070022496 |
| | | WO 2000/063432 |
| | | WO 2004/049786 |
| C-9 | Erwinia resistance | WO 2004/049786 |
| C10 | Closterovirus resistance | WO 2007/073167 |
| | | WO 2007/053015 |
| | | WO 2002/022836 |
| C-11 | Tobamovirus resistance | WO 2006/038794 |

In one embodiment of the invention, the plants comprising or expressing traits of C-1 to C-11 of Table C, in total, or parts thereof, or propagation material of said plant are treated or contacted with Fluopyram alone, or in the form of compositions comprising Fluopyram.

**Table D**

| Non-exhaustive list of transgenic events and traits the invention can be worked on with reference to patent applications. | | | | |
|---|---|---|---|---|
| **No.** | **Plant species** | **Transgenic event** | **Trait** | **Patent reference** |
| D-1 | Corn | PV-ZMGT32 (NK603) | Glyphosate tolerance | US 2007-056056 |
| D-2 | Corn | MIR604 | Insect resistance (Cry3a055) | EP 1 737 290 |
| D-3 | Corn | LY038 | High lysine content | US 7,157,281 |
| D-4 | Corn | 3272 | Self processing corn (alpha-amylase) | US 2006-230473 |
| D-5 | Corn | PV-ZMIR13 (MON863) | Insect resistance (Cry3Bb) | US 2006-095986 |
| D-6 | Corn | DAS-59122-7 | Insect resistance (Cry34Ab1/Cry35Ab1) | US 2006-070139 |
| D-7 | Corn | TC1507 | Insect resistance (Cry1F) | US 7,435,807 |
| D-8 | Corn | MON810 | Insect resistance (Cry1Ab) | US 2004-180373 |
| D-9 | Corn | VIP1034 | Insect resistance | WO 03/052073 |
| D-10 | Corn | B16 | Glufosinate resistance | US 2003-126634 |
| D-11 | Corn | GA21 | Glyphosate resistance | US 6,040,497 |
| D-12 | Corn | GG25 | Glyphosate resistance | US 6,040,497 |
| D-13 | Corn | GJ11 | Glyphosate resistance | US 6,040,497 |
| D-14 | Corn | FI117 | Glyphosate resistance | US 6,040,497 |
| D-15 | Corn | GAT-ZM1 | Glufosinate tolerance | WO 01/51654 |
| D-16 | Corn | DP-098140-6 | Glyphosate tolerance / ALS inhibitor tolerance | WO 2008/112019 |
| D-17 | Wheat | Event 1 | Fusarium resistance (trichothecene 3-O-acetyltransferase) | CA 2561992 |
| D-18 | Sugar beet | T227-1 | Glyphosate tolerance | US 2004-117870 |
| D-19 | Sugar beet | H7-1 | Glyphosate tolerance | WO 2004-074492 |
| D-20 | Soybean | MON89788 | Glyphosate tolerance | US 2006-282915 |
| D-21 | Soybean | A2704-12 | Glufosinate tolerance | WO 2006/108674 |
| D-22 | Soybean | A5547-35 | Glufosinate tolerance | WO 2006/108675 |
| D-23 | Soybean | DP-305423-1 | High oleic acid / ALS inhibitor tolerance | WO 2008/054747 |
| D-24 | Rice | GAT-OS2 | Glufosinate tolerance | WO 01/83818 |
| D-25 | Rice | GAT-OS3 | Glufosinate tolerance | US 2008-289060 |
| D-26 | Rice | PE-7 | Insect resistance (Cry1Ac) | WO 2008/114282 |
| D-27 | Oilseed rape | MS-B2 | Male sterility | WO 01/31042 |
| D-28 | Oilseed rape | MS-BN1/RF- BN1 | Male sterility/restoration | WO 01/41558 |
| D-29 | Oilseed rape | RT73 | Glyphosate resistance | WO 02/36831 |
| D-30 | Cotton | CE43-67B | Insect resistance (Cry1Ab) | WO 2006/128573 |
| D-31 | Cotton | CE46-02A | Insect resistance (Cry1Ab) | WO 2006/128572 |
| D-32 | Cotton | CE44-69D | Insect resistance (Cry1Ab) | WO 2006/128571 |
| D-33 | Cotton | 1143-14A | Insect resistance (Cry1Ab) | WO 2006/128569 |
| D-34 | Cotton | 1143-51B | Insect resistance (Cry1Ab) | WO 2006/128570 |
| D-35 | Cotton | T342-142 | Insect resistance (Cry1Ab) | WO 2006/128568 |
| D-36 | Cotton | event3006- 210-23 | Insect resistance (Cry1Ac) | WO 2005/103266 |
| D-37 | Cotton | PV-GHGT07 (1445) | Glyphosate tolerance | US 2004-148666 |
| D-38 | Cotton | MON88913 | Glyphosate tolerance | WO 2004/072235 |
| D-39 | Cotton | EE-GH3 | Glyphosate tolerance | WO 2007/017186 |
| D-40 | Cotton | T304-40 | Insect-resistance (Cry1Ab) | WO2008/122406 |
| D-41 | Cotton | Cot202 | Insect resistance (VIP3) | US 2007-067868 |
| D-42 | Cotton | LLcotton25 | Glufosinate resistance | WO 2007/017186 |
| D-43 | Cotton | EE-GH5 | Insect resistance (Cry1Ab) | WO 2008/122406 |
| D-44 | Cotton | event 281-24- 236 | Insect resistance (Cry1F | WO 2005/103266 |
| D-45 | Cotton | Cot102 | Insect resistance (Vip3A) | US 2006-130175 |
| D-46 | Cotton | MON 15985 | Insect resistance (Cry1A/Cry2Ab) | US 2004-250317 |
| D-47 | Bent Grass | Asr-368 | Glyphosate tolerance | US 2006-162007 |
| D-48 | Brinjal | EE-1 | Insect resistance (Cry1Ac) | WO 2007/091277 |

In one embodiment of the invention, the plants comprising a transgenic event or expressing a trait of D-1 to D-48 of Table D, in total, or parts thereof, or propagation material of said plant are treated or contacted with Fluopyram alone, or in the form of compositions comprising Fluopyram.

In one embodiment, the compositions comprising Fluopyram, contain another active ingredient. In particular this can be a fungicide or an acaricide, a nematicide, or an insecticide, or a herbicidal safener.

Typically, the weight ratio between Fluopyram and another active ingredient is between 1000 to 1 and 1 to 125, preferably between 125 to 1 and 1 to 50 und particularly preferred between 25 to 1 and 1 to 5.

Preferred are the following fungicides selected from the group consisting of:
F1) a compound capable to inhibit the nucleic acid synthesis like benalaxyl, benalaxyl-M, bupirimate, clozylacon, dimethirimol, ethirimol, furalaxyl, hymexazol, mefenoxam, metalaxyl, metalaxyl-M, ofurace, oxadixyl, oxolinic acid ;
F2) a compound capable to inhibit the mitosis and cell division like benomyl, carbendazim, diethofencarb, ethaboxam, fuberidazole, pencycuron, thiabendazole, thiophanate-methyl, zoxamide;
F3) a compound capable to inhibit the respiration for example
   as CI-respiration inhibitor like diflumetorim ;
   as CII-respiration inhibitor like boscalid, carboxin, fenfuram, flutolanil, furametpyr, furmecyclox, mepronil, oxycarboxin, penthiopyrad, thifluzamide ;
   as CIII-respiration inhibitor like amisulbrom, azoxystrobin, cyazofamid, dimoxystrobin, enestrobin, famoxadone, fenamidone, fluoxastrobin, kresoxim-methyl, metominostrobin, orysastrobin, picoxystrobin, pyraclostrobin, trifloxystrobin ;
F4) a compound capable of to act as an uncoupler like dinocap, fluazinam, meptyldinocap;
F5) a compound capable to inhibit ATP production like fentin acetate, fentin chloride, fentin hydroxide, silthiofam;
F6) a compound capable to inhibit AA and protein biosynthesis like andoprim, blasticidin-S, cyprodinil, kasugamycin, kasugamycin hydrochloride hydrate, mepanipyrim, pyrimethanil;
F7) a compound capable to inhibit the signal transduction like fenpiclonil, fludioxonil, quinoxyfen;
F8) a compound capable to inhibit lipid and membrane synthesis like biphenyl, chlozolinate, edifenphos, etridiazole, iodocarb, iprobenfos, iprodione, isoprothiolane, procymidone, propamocarb, propamocarb hydrochloride, pyrazophos, tolclofos-methyl, vinclozolin ;
F9) a compound capable to inhibit ergosterol biosynthesis like aldimorph, azaconazole, bitertanol, bromuconazole, cyproconazole, diclobutrazole, difenoconazole, diniconazole, diniconazole-M, dodemorph, dodemorph acetate, epoxiconazole, etaconazole, fenarimol, fenbuconazole, fenhexamid, fenpropidin, fenpropimorph, fluquinconazole, flurprimidol, flusilazole, flutriafol, furconazole, furconazole-cis, hexaconazole, imazalil, imazalil sulfate, imibenconazole, ipconazole, metconazole, myclobutanil, naftifine, nuarimol, oxpoconazole, paclobutrazol, pefurazoate, penconazole, prochloraz, propiconazole, prothioconazole, pyributicarb, pyrifenox, simeconazole, spiroxamine, tebuconazole, terbinafine, tetraconazole, triadimefon, triadimenol, tridemorph, triflumizole, triforine, triticonazole, uniconazole, viniconazole, voriconazole ;
F10) a compound capable to inhibit cell wall synthesis like benthiavalicarb, dimethomorph, flumorph, iprovalicarb, mandipropamid, polyoxins, polyoxorim, validamycin A;
F11) a compound capable to inhibit melanine biosynthesis like carpropamid, diclocymet, fenoxanil, phthalide, pyroquilon, tricyclazole;
F12) a compound capable to induce a host defence like acibenzolar-S-methyl, probenazole, tiadinil;
F13) a compound capable to have a multisite action like Bordeaux mixture, captafol, captan, chlorothalonil, copper naphthenate, copper oxide, copper oxychloride, copper preparations such as copper hydroxide, copper sulphate, dichlofluanid, dithianon, dodine, dodine free base, ferbam, fluorofolpet, folpet, guazatine, guazatine acetate, iminoctadine, iminoctadine albesilate, iminoctadine triacetate, mancopper, mancozeb, maneb, metiram, metiram zinc, oxine-copper, propineb, sulphur and sulphur preparations including calcium polysulphide, thiram, tolylfluanid, zineb, ziram ;
F14) a compound selected in the following list: (2E)-2-(2-{[6-(3-chloro-2-methylphenoxy)-5-fluoropyrimidin-4-yl]oxy}phenyl)-2-(methoxyimino)-N-methylacetamide, (2E)-2-{2-[({[(1E)-1-(3-{[(E)-1-fluoro-2-phenylvinyl]oxy}phenyl)ethylidene]amino}oxy)methyl]phenyl}-2-(methoxyimino)-N-methylacetamide, 1-(4-chlorophenyl)-2-(1H-1,2,4-triazol-1-yl)cycloheptanol, 1-[(4-methoxyphenoxy)methyl]-2,2-dimethylpropyl-1H-imidazole-1-carboxylate,1-methyl-N-[2-(1,1,2,2-tetrafluoroethoxy)phenyl]-3-(trifluoromethyl)-1H-pyrazole-4-carboxamide, 2,3,5,6-tetrachloro-4-(methylsulfonyl)pyridine, 2-butoxy-6-iodo-3-propyl-4H-chromen-4-one, 2-chloro-N-(1,1,3-trimethyl-2,3-dihydro-1H-inden-4-yl)nicotinamide, 2-phenylphenol and salts, 3-(difluoromethyl)-1-methyl-N-[2-(1,1,2,2-tetrafluoroethoxy)phenyl]-1H-pyrazole-4-carboxamide,3-(difluoromethyl)-N-[(9R)-9-isopropyl-1,2,3,4-tetrahydro-1,4-methanonaphthalen-5-yl]-1-methyl-1H-pyrazole-4-carboxamide, 3-(difluoromethyl)-N-[(9S)-9-isopropyl-1,2,3,4-tetrahydro-1,4-methanonaphthalen-5-yl]-1-methyl-1H-pyrazole-4-carboxamide, 3-(difluoromethyl)-N-[4'-(3,3-dimethylbut-1-yn-1-yl)biphenyl-2-yl]-1-methyl-1H-pyrazole-4-carboxamide, 3,4,5-trichloropyridine-2,6-dicarbonitrile, 3-[5-(4-chlorophenyl)-2,3-dimethylisoxazolidin-3-yl]pyridine, 3-chloro-5-(4-chlorophenyl)-4-(2,6-difluorophenyl)-6-methylpyridazine , 4-(4-chlorophenyl)-5-(2,6-difluorophenyl)-3,6-dimethylpyridazine, 5-chloro-7-(4-methylpiperidin-1-yl)-6-(2,4,6-trifluorophenyl)[1,2,4]triazolo[1,5-a]pyrimidine, 8-hydroxyquinoline sulfate, benthiazole, bethoxazin, capsimycin, carvone, chinomethionat, cufraneb, cyflufenamid, cymoxanil, dazomet, debacarb, dichlorophen, diclomezine, dicloran, difenzoquat, difenzoquat methylsulphate, diphenylamine, ecomate, ferimzone, flumetover, fluopicolide, fluoroimide, flusulfamide, fosetyl-aluminium, fosetyl-calcium, fosetyl-sodium, hexachlorobenzene, irumamycin, isotianil, methasulfocarb, methyl (2E)-2-{2-[({cyclopropyl[(4-methoxyphenyl)imino]methyl}thio)methyl]phenyl}-3-methoxyacrylate, methyl 1-(2,2-dimethyl-2,3-dihydro-1H-inden-1-yl)-1H-imidazole-5-carboxylate, methyl isothiocyanate, metrafenone, mildiomycin, N-(3',4'-dichloro-5-fluorobiphenyl-2-yl)-3-(difluoromethyl)-1-methyl-1H-pyrazole-4-carboxamide, N-(3-ethyl-3,5,5-trimethylcyclohexyl)-3-(formylamino)-2-hydroxybenzamide, N-(4-chloro-2-nitrophenyl)-N-ethyl-4-methylbenzenesulfonamide, N-(4-chlorobenzyl)-3-[3-methoxy-4-(prop-2-yn-1-yloxy)phenyl]propanamide, N-[(4-chlorophenyl)(cyano)methyl]-3-[3-methoxy-4-(prop-2-yn-1-yloxy)phenyl]propanamide, N-[(5-bromo-3-chloropyridin-2-yl)methyl]-2,4-dichloronicotinamide, N-[1-(5-bromo-3-chloropyridin-2-yl)ethyl]-2,4-dichloronicotinamide, N-[1-(5-bromo-3-chloropyridin-2-yl)ethyl]-2-fluoro-4-iodonicotinamide, N-[2-(1,3-dimethylbutyl)phenyl]-5-fluoro-1,3-dimethyl-1H-pyrazole-4-carboxamide, N-{(Z)-[(cyclopropylmethoxy)imino][6-(difluoromethoxy)-2,3-difluorophenyl]methyl}-2-phenylacetamide, N-{2-[1,1'-bi(cyclopropyl)-2-yl]phenyl} -3-(difluoromethyl)-1-methyl-1H-pyrazole-4-carboxamide, N-{2-[3-chloro-5-(trifluoromethyl)pyridin-2-yl]ethyl}-2-(trifluoromethyl)benzamide, natamycin, N-ethyl-N-methyl-N'-{2-methyl-5-(trifluoromethyl)-4-[3-(trimethylsilyl)propoxy]phenyl imidoformamide, N-ethyl-N-methyl-N'-{2-methyl-5-(difluoromethyl)-4-[3-(trimethylsilyl)propoxy]phenyl}imidoformamide, nickel dimethyldithiocarbamate, nitrothal-isopropyl, O-{1-[(4-methoxyphenoxy)methyl]-2,2-dimethylpropyl}1H-imidazole-1-carbothioate, octhilinone, oxamocarb, oxyfenthiin, pentachlorophenol and salts, phosphorous acid and its salts, piperalin, propamocarb fosetylate, propanosine-sodium, proquinazid, pyribencarb, pyrrolnitrine, quintozene, S-allyl-5-amino-2-isopropyl-4-(2-methylphenyl)-3-oxo-2,3-dihydro-1H-pyrazole-1-carbothioate, tecloftalam, tecnazene, triazoxide, trichlamide, valiphenal, zarilamid.

Particularly preferred fungicides as additional actives ingredients are the following fungicides selected from the group consisting of: azoxystrobin, dimoxystrobin, kresoxim-methyl, orysastrobin, pyraclostrobin, trifloxystrobin, bixafen, boscalid, isopyrazam, metalaxyl, penthiopyrad, 3-difluoromethyl-1-methyl-1 H-pyrazole-4-carboxylic acid (2',4',5'-trifluorobiphenyl-2-yl)-amide, N-(2-bicyclopropyl-2-yl-phenyl)-3-difluoromethyl-1-methyl-1H-pyrazole-4-carboxylic acid amide, dimethomorph, fluopicolide, difenoconazole, ipconazole, epoxiconazole, fluquinconazole, flusilazole, flutriafol, metconazol, myclobutanil, propiconazole, prothioconazole, tebuconazole, tetraconazole, triticonazole, prochloraz, carbendazim, fluazinam, cyprodinil, pyrimethanil, fludioxonil, dodemorph, fenpropimorph, tridemorph, fenpropidin, iprodione, vinclozolin, famoxadone, probenazole, captan, folpet, 5-ethyl-6-octyl- [1,2,4]triazolo[1,5-a]pyrimidine-7-ylamine, mancozeb, maneb, metiram, thiram, dithianon, fosetyl, fosetyl-aluminium, chlorothalonil, thiophanate methyl, cymoxanil, metrafenone, spiroxamine, bixafen, N-(3',4',5'-trifluorobiphenyl-2-yl)- 3-difluoromethyl- 1 -methyl-1 H-pyrazole-4-carboxamide, N-[2-(4'-trifluoromethylthio)-biphenyl]-3-difluoromethyl-1 -methyl-1 H-pyrazole-4-carboxamide,N-[2-(1,3-dimethylbutyl)-phenyl]-1,3-dimethyl-5-fluoro-1 H-pyrazole-4-carboxamide, N-(2-bicyclopropyl-2-yl-phenyl)-3-difluoromethyl-1-methyl-1H-pyrazole-4-carboxamide,N-(cis-2-bicyclopropyl-2-yl-phenyl)-3-difluoromethyl-1 -methyl-1 H-pyrazole-4-carboxamide, N-(trans-2-bicyclopropyl-2-yl-phenyl)-3-difluoromethyl-1-methyl-1 H-pyrazole-4-carboxamide , N-[1,2,3,4-tetrahydro-9-(1 -methylethyl)-1,4-methanonaphthalen-5-yl]-3-(difluoromethyl)-1 -methyl-1 H-pyrazole-4-carboxamide.

Preferred are the following Insecticides/acaricides/nematicides selected from the group consisting of:
(1) Acetylcholinesterase (AChE) inhibitors, for example carbamates, e.g. alanycarb, aldicarb, aldoxycarb, allyxycarb, aminocarb, bendiocarb, benfuracarb, bufencarb, butacarb, butocarboxim, butoxycarboxim, carbaryl, carbofuran, carbosulfan, cloethocarb, dimetilan, ethiofencarb, fenobucarb, fenothiocarb, formetanate, furathiocarb, isoprocarb, metam-sodium, methiocarb, methomyl, metolcarb, oxamyl, pirimicarb, promecarb, propoxur, thiodicarb, thiofanox, trimethacarb, XMC, and xylylcarb; or organophosphates, e.g. acephate, azamethiphos, azinphos (-methyl, -ethyl), bromophos-ethyl, bromfenvinfos (-methyl), butathiofos, cadusafos, carbophenothion, chlorethoxyfos, chlorfenvinphos, chlormephos, chlorpyrifos (-methyl/-ethyl), coumaphos, cyanofenphos, cyanophos, chlorfenvinphos, demeton-S-methyl, demeton-S-methylsulphon, dialifos, diazinon, dichlofenthion, dichlorvos/DDVP, dicrotophos, dimethoate, dimethylvinphos, dioxabenzofos, disulfoton, EPN, ethion, ethoprophos, etrimfos, famphur, fenamiphos, fenitrothion, fensulfothion, fenthion, flupyrazofos, fonofos, formothion, fosmethilan, fosthiazate, heptenophos, iodofenphos, iprobenfos, isazofos, isofenphos, isopropyl, O-salicylate, isoxathion, malathion, mecarbam, methacrifos, methamidophos, methidathion, mevinphos, monocrotophos, naled, omethoate, oxydemeton-methyl, parathion (-methyl/-ethyl), phenthoate, phorate, phosalone, phosmet, phosphamidon, phosphocarb, phoxim, pirimiphos (-methyl/-ethyl), profenofos, propaphos, propetamphos, prothiofos, prothoate, pyraclofos, pyridaphenthion, pyridathion, quinalphos, sebufos, sulfotep, sulprofos, tebupirimfos, temephos, terbufos, tetrachlorvinphos, thiometon, triazophos, triclorfon, vamidothion, and imicyafos.
(2) GABA-gated chloride channel antagonists, for example organochlorines, e.g. camphechlor, chlordane, endosulfan, gamma-HCH, HCH, heptachlor, lindane, and methoxychlor; or fiproles (phenylpyrazoles), e.g. acetoprole, ethiprole, fipronil, pyrafluprole, pyriprole, and vaniliprole.
(3) Sodium channel modulators/voltage-dependent sodium channel blockers, for example pyrethroids, e.g. acrinathrin, allethrin (d-cis-trans, d-trans), beta-cyfluthrin, bifenthrin, bioallethrin, bioallethrin S-cyclopentyl isomer, bioethanomethrin, biopermethrin, bioresmethrin, chlovaporthrin, cis-cypermethrin, cis-resmethrin, cis-permethrin, clocythrin, cycloprothrin, cyfluthrin, cyhalothrin, cypermethrin (alpha-, beta-, theta-, zeta-), cyphenothrin, deltamethrin, empenthrin (1R isomer), esfenvalerate, etofenprox, fenfluthrin, fenpropathrin, fenpyrithrin, fenvalerate, flubrocythrinate, flucythrinate, flufenprox, flumethrin, fluvalinate, fubfenprox, gamma-cyhalothrin, imiprothrin, kadethrin, lambda-cyhalothrin, metofluthrin, permethrin (cis-, trans-), phenothrin (1R trans isomer), prallethrin, profluthrin, protrifenbute, pyresmethrin, resmethrin, RU 15525, silafluofen, tau-fluvalinate, tefluthrin, terallethrin, tetramethrin (-1R- isomer), tralomethrin, transfluthrin, ZXI 8901, pyrethrin (pyrethrum), eflusilanat;
   DDT; or methoxychlor.
(4) Nicotinergic acetylcholine receptor agonists/antagonists, for example chloronicotinyls, e.g. acetamiprid, clothianidin, dinotefuran, imidacloprid, imidaclothiz, nitenpyram, nithiazine, thiacloprid, thiamethoxam, AKD-1022, nicotine, bensultap, cartap, thiosultap-sodium, and thiocylam.
(5) Allosteric acetylcholine receptor modulators (agonists), for example spinosyns, e.g. spinosad and spinetoram.
(6) Chloride channel activators, for example mectins/macrolides, e.g. abamectin, emamectin, emamectin benzoate, ivermectin, lepimectin, and milbemectin; or juvenile hormone analogues, e.g. hydroprene, kinoprene, methoprene, epofenonane, triprene, fenoxycarb, pyriproxifen, and diofenolan.
(7) Active ingredients with unknown or non-specific mechanisms of action, for example gassing agents, e.g. methyl bromide, chloropicrin and sulfuryl fluoride; selective antifeedants, e.g. cryolite, pymetrozine, pyrifluquinazon and flonicamid; or mite growth inhibitors, e.g. clofentezine, hexythiazox, etoxazole.
(8) Oxidative phosphorylation inhibitors, ATP disruptors, for example diafenthiuron; organotin compounds, e.g. azocyclotin, cyhexatin and fenbutatin oxide; or propargite, tetradifon.
(9) Oxidative phoshorylation decouplers acting by interrupting the H proton gradient, for example chlorfenapyr, binapacryl, dinobuton, dinocap and DNOC.
(10) Microbial disruptors of the insect gut membrane, for example *Bacillus thuringiensis* strains.
(11) Chitin biosynthesis inhibitors, for example benzoylureas, e.g. bistrifluron, chlorfluazuron, diflubenzuron, fluazuron, flucycloxuron, flufenoxuron, hexaflumuron, lufenuron, novaluron, noviflumuron, penfluron, teflubenzuron or triflumuron.
(12) Buprofezin.
(13) Moulting disruptors, for example cyromazine.
(14) Ecdysone agonists/disruptors, for example diacylhydrazines, e.g. chromafenozide, halofenozide, methoxyfenozide, tebufenozide, and Fufenozide (JS118); or azadirachtin.
(15) Octopaminergic agonists, for example amitraz.
(16) Site III electron transport inhibitors/site II electron transport inhibitors, for example hydramethylnon; acequinocyl; fluacrypyrim; or cyflumetofen and cyenopyrafen.
(17) Electron transport inhibitors, for example Site I electron transport inhibitors, from the group of the METI acaricides, e.g. fenazaquin, fenpyroximate, pyrimidifen, pyridaben, tebufenpyrad, tolfenpyrad, and rotenone; or voltage-dependent sodium channel blockers, e.g. indoxacarb and metaflumizone.
(18) Fatty acid biosynthesis inhibitors, for example tetronic acid derivatives, e.g. spirodiclofen and spiromesifen; or tetramic acid derivatives, e.g. spirotetramat.
(19) Neuronal inhibitors with unknown mechanism of action, e.g. bifenazate.
(20) Ryanodine receptor effectors, for example diamides, e.g. flubendiamide, (R),(S)-3-chloro-N¹{2-methyl-4-[1,2,2,2-tetrafluoro-1-(trifluoromethyl)ethyl]phenyl}-N²-(1-methyl-2-methylsulphonylethyl)phthalamide, chlorantraniliprole (Rynaxypyr), or Cyantraniliprole (Cyazypyr).
(21) Further active ingredients with unknown mechanism of action, for example amidoflumet, benclothiaz, benzoximate, bromopropylate, buprofezin, chinomethionat, chlordimeform, chlorobenzilate, clothiazoben, cycloprene, dicofol, dicyclanil, fenoxacrim, fentrifanil, flubenzimine, flufenerim, flutenzin, gossyplure, japonilure, metoxadiazone, petroleum, potassium oleate, pyridalyl, sulfluramid, tetrasul, triarathene or verbutine; or one of the following known active compounds 4-{[(6-brompyrid-3-yl)methyl](2-fluorethyl)amino}furan-2(5H)-on (known from WO 2007/115644), 4-{[(6-fluorpyrid-3-yl)methyl](2,2-difluorethyl)amino}furan-2(5H)-on (known from WO 2007/115644), 4-{[(2-chlor-1,3-thiazol-5-yl)methyl](2-fluorethyl)amino}furan-2(5H)-on (known from WO 2007/115644), 4-{[(6-chlorpyrid-3-yl)methyl](2-fluorethyl)amino}furan-2(5H)-on (known from WO 2007/ 115644), 4-{[(6-chlorpyrid-3-yl)methyl](2,2-difluorethyl)amino}furan-2(5H)-on known from WO 2007/115644), 4-{[(6-chlor-5-fluorpyrid-3-yl)methyl](methyl)amino}furan-2(5H)-on (known from WO 2007/115643), 4-{[(5,6-dichlorpyrid-3-yl)methyl](2-fluorethyl)amino}furan-2(5H)-on (known from WO 2007/115646), 4-{[(6-chlor-5-fluorpyrid-3-yl)methyl](cyclopropyl)amino}furan-2(5H)-on (known from WO 2007/115643), 4-{[(6-chlorpyrid-3-yl)methyl](cyclopropyl)amino}furan-2(5H)-on (known from EP-A-0 539 588), 4-{[(6-chlorpyrid-3-yl)methyl](methyl)amino}furan-2(5H)-on (known from EP-A-0 539 588), [(6-chlorpyridin-3-yl)methyl](methyl)oxido-λ⁴-sulfanylidencyanamid (known from WO 2007/149134), [1-(6-chlorpyridin-3-yl)ethyl](methyl)oxido-λ⁴-sulfanylidencyanamid (known from WO 2007/149134) and its diastereomeres (A) and (B) 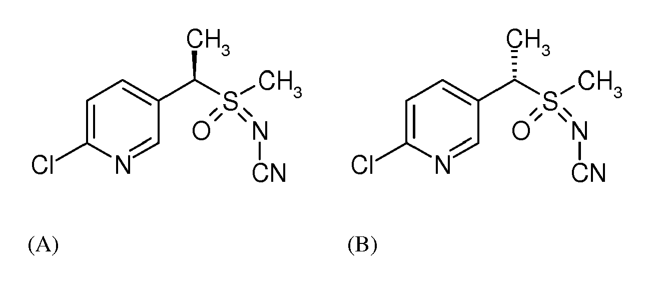 (also known from WO 2007/149134), [(6-trifluormethylpyridin-3-yl)methyl](methyl)oxido-λ⁴-sulfanylidencyanamid (known from WO 2007/095229), or [1-(6-trifluormethylpyridin-3-yl)ethyl](methyl)oxido-λ⁴-sulfanylidencyanamid (known from WO 2007/149134) and its diastereomeres (C) and (D), namely Sulfoxaflor 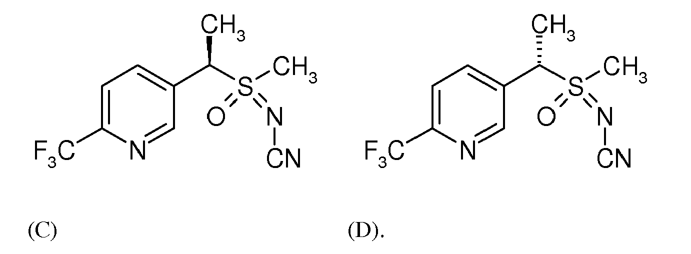 Particularly preferred acaricides, nematicides, or insecticides as additional active ingredients to Fluopyram are selected from the group consisting of acephate, chlorpyrifos, diazinon, dichlorvos, dimethoate, fenitrothion, methamidophos, methidathion, methyl-parathion, monocrotophos, phorate, profenofos, terbufos, aldicarb, carbaryl, carbofuran, carbosulfan, methomyl, thiodicarb, bifenthrin, cyfluthrin, cypermethrin, alpha-cypermethrin, zeta-cypermethrin, deltamethrin, esfenvalerate, lambda-cyhalothrin, permethrin, tefluthrin, diflubenzuron, flufenoxuron, lufenuron, teflubenzuron, spirotetramat; clothianidin, dinotefuran, imidacloprid, thiamethoxam, acetamiprid, thiacloprid; endosulfan, fipronil, abamectin, emamectin, spinosad, spinetoram, hydramethylnon; chlorfenapyr; fenbutatin oxide, indoxacarb, metaflumizone, flonicamid, flubendiamide, chlorantraniliprole, cyazypyr (HGW86), cyflumetofen.

Very particulary preferred acaricides, nematicides, or insecticides as additional active ingredients to Fluopyram are selected from the group consisting of thiodicarb, cyfluthrin, tefluthrin, clothianidin, imidacloprid, thiamethoxam, acetamiprid, thiacloprid; fipronil, abamectin, flubendiamide, chlorantraniliprole, cyazypyr

### Foliar Fungicidal

Preferably, Fluopyram is applied as a composition further comprising an agriculturally acceptable support, carrier or filler.

According to the invention, the term "support" denotes a natural or synthetic, organic or inorganic compound with which the active compound of formula (I) is combined or associated to make it easier to apply, notably to the parts of the plant. This support is thus generally inert and should be agriculturally acceptable. The support may be a solid or a liquid. Examples of suitable supports include clays, natural or synthetic silicates, silica, resins, waxes, solid fertilisers, water, alcohols, in particular butanol, organic solvents, mineral and plant oils and derivatives thereof. Mixtures of such supports may also be used.

The composition according to the invention may also comprise additional components. In particular, the composition may further comprise a surfactant. The surfactant can be an emulsifier, a dispersing agent or a wetting agent of ionic or non-ionic type or a mixture of such surfactants. Mention may be made, for example, of polyacrylic acid salts, lignosulphonic acid salts, phenolsulphonic or naphthalenesulphonic acid salts, polycondensates of ethylene oxide with fatty alcohols or with fatty acids or with fatty amines, substituted phenols (in particular alkylphenols or arylphenols), salts of sulphosuccinic acid esters, taurine derivatives (in particular alkyl taurates), phosphoric esters of polyoxyethylated alcohols or phenols, fatty acid esters of polyols, and derivatives of the present compounds containing sulphate, sulphonate and phosphate functions. The presence of at least one surfactant is generally essential when the active compound and / or the inert support are water-insoluble and when the vector agent for the application is water. Preferably, surfactant content may be comprised from 5% to 40% by weight of the composition.

Colouring agents such as inorganic pigments, for example iron oxide, titanium oxide, ferrocyanblue, and organic pigments such as alizarin, azo and metallophthalocyanine dyes, and trace elements such as iron, manganese, boron, copper, cobalt, molybdenum and zinc salts can be used.

Optionally, other additional components may also be included, e.g. protective colloids, adhesives, thickeners, thixotropic agents, penetration agents, stabilisers, sequestering agents. More generally, the active compounds can be combined with any solid or liquid additive, which complies with the usual formulation techniques.

In general, the composition according to the invention may contain from 0.05 to 99% by weight of active compounds, preferably from 10 to 70% by weight.

The combination or composition according to the invention can be used as such, in form of their formulations or as the use forms prepared therefrom, such as aerosol dispenser, capsule suspension, cold fogging concentrate, dustable powder, emulsifiable concentrate, emulsion oil in water, emulsion water in oil, encapsulated granule, fine granule, flowable concentrate for seed treatment, gas (under pressure), gas generating product, granule, hot fogging concentrate, macrogranule, microgranule, oil dispersible powder, oil miscible flowable concentrate, oil miscible liquid, paste, plant rodlet, powder for dry seed treatment, seed coated with a pesticide, soluble concentrate, soluble powder, solution for seed treatment, suspension concentrate (flowable concentrate), ultra low volume (ULV) liquid, ultra low volume (ULV) suspension, water dispersible granules or tablets, water dispersible powder for slurry treatment, water soluble granules or tablets, water soluble powder for seed treatment and wettable powder.

The treatment of plants and plant parts with the active compound combination according to the invention is carried out directly or by action on their environment, habitat or storage area by means of the normal treatment methods, for example by watering (drenching), drip irrigation, spraying, atomizing, broadcasting, dusting, foaming, spreading-on, and as a powder for dry seed treatment, a solution for seed treatment, a water-soluble powder for seed treatment, a water-soluble powder for slurry treatment, or by encrusting.

These compositions include not only compositions which are ready to be applied to the plant or seed to be treated by means of a suitable device, such as a spraying or dusting device, but also concentrated commercial compositions which must be diluted before application to the crop.

The active compounds within the composition according to the invention have potent microbicide activity and can be employed for controlling undesired micro-organisms, such as fungi or bacteria, in crop protection or in the protection of materials.

Within the composition according to the invention, fungicide compounds can be employed in crop protection for example for controlling Plasmodiophoromycetes, Oomycetes, Chytridiomycetes, Zygomycetes, Ascomycetes, Basidiomycetes and Deuteromycetes.

Within the composition according to the invention, bactericide compounds can be employed in crop protection for example for controlling Pseudomonadaceae, Rhizobiaceae, Enterobacteriaceae, Corynebacteriaceae and Streptomycetaceae.

The fungicide composition according to the invention can be used to curatively or preventively control the phytopathogenic fungi of plants or crops. Thus, according to a further aspect of the invention, there is provided a method for curatively or preventively controlling the phytopathogenic fungi of plants or crops comprising the use of a fungicide composition according to the invention by application to the seed, the plant or to the fruit of the plant or to the soil in which the plant is growing or in which it is desired to grow.

### Insecticide Foliar

The methods and compositions according to the invention can be used to control the following animal pests.

From the order of the Anoplura (Phthiraptera), for example, Damalinia spp., Haematopinus spp., Linognathus spp., Pediculus spp., Trichodectes spp.

From the class of the Arachnida, for example, Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

From the class of the Bivalva, for example, Dreissena spp.

From the order of the Chilopoda, for example, Geophilus spp., Scutigera spp.

From the order of the Coleoptera, for example, Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.

From the order of the Collembola, for example, Onychiurus armatus.

From the order of the Dermaptera, for example, Forficula auricularia.

From the order of the Diplopoda, for example, Blaniulus guttulatus.

From the order of the Diptera, for example, Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp., Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

From the class of the Gastropoda, for example, Arion spp., Biomphalaria spp., Bulinus spp., Deroceras spp., Galba spp., Lymnaea spp., Oncomelania spp., Succinea spp.

From the class of the helminths, for example, Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

It is furthermore possible to control protozoa, such as Eimeria.

From the order of the Heteroptera, for example, Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

From the order of the Homoptera, for example, Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

From the order of the Hymenoptera, for example, Diprion spp., Hoplocampa spp., Lasius spp., Monomorium pharaonis, Vespa spp.

From the order of the Isopoda, for example, Armadillidium vulgare, Oniscus asellus, Porcellio scaber.

From the order of the Isoptera, for example, Reticulitermes spp., Odontotermes spp.

From the order of the Lepidoptera, for example, Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.

From the order of the Orthoptera, for example, Acheta domesticus, Blatta orientalis, Blattella germanica, Gryllotalpa spp., Leucophaea maderae, Locusta spp., Melanoplus spp., Periplaneta americana, Schistocerca gregaria.

From the order of the Siphonaptera, for example, Ceratophyllus spp., Xenopsylla cheopis.

From the order of the Symphyla, for example, Scutigerella immaculata.

From the order of the Thysanoptera, for example, Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp.

From the order of the Thysanura, for example, Lepisma saccharina.

The phytoparasitic nematodes include, for example, Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp.

If appropriate, the compounds according to the invention can, at certain concentrations or application rates, also be used as herbicides, safeners, growth regulators or agents to improve plant properties, or as microbicides, for example as fungicides, antimycotics, bactericides, viricides (including agents against viroids) or as agents against MLO (Mycoplasma-like organisms) and RLO (Rickettsia-like organisms). If appropriate, they can also be employed as intermediates or precursors for the synthesis of other active compounds.

The active compounds can be converted to the customary formulations, such as solutions, emulsions, wettable powders, water- and oil-based suspensions, powders, dusts, pastes, soluble powders, soluble granules, granules for broadcasting, suspension-emulsion concentrates, natural materials impregnated with active compound, synthetic materials impregnated with active compound, fertilizers and microencapsulations in polymeric substances.

These formulations are produced in a known manner, for example by mixing the active compounds with extenders, that is liquid solvents and/or solid carriers, optionally with the use of surfactants, that is emulsifiers and/or dispersants and/or foam-formers. The formulations are prepared either in suitable plants or else before or during the application.

Suitable for use as auxiliaries are substances which are suitable for imparting to the composition itself and/or to preparations derived therefrom (for example spray liquors, seed dressings) particular properties such as certain technical properties and/or also particular biological properties. Typical suitable auxiliaries are: extenders, solvents and carriers.

Suitable extenders are, for example, water, polar and non-polar organic chemical liquids, for example from the classes of the aromatic and non-aromatic hydrocarbons (such as paraffins, alkylbenzenes, alkylnaphthalenes, chlorobenzenes), the alcohols and polyols (which, if appropriate, may also be substituted, etherified and/or esterified), the ketones (such as acetone, cyclohexanone), esters (including fats and oils) and (poly)ethers, the unsubstituted and substituted amines, amides, lactams (such as *N*-alkylpyrrolidones) and lactones, the sulphones and sulphoxides (such as dimethyl sulphoxide).

If the extender used is water, it is also possible to employ, for example, organic solvents as auxiliary solvents. Essentially, suitable liquid solvents are: aromatics such as xylene, toluene or alkylnaphthalenes, chlorinated aromatics and chlorinated aliphatic hydrocarbons such as chlorobenzenes, chloroethylenes or methylene chloride, aliphatic hydrocarbons such as cyclohexane or paraffins, for example petroleum fractions, mineral and vegetable oils, alcohols such as butanol or glycol and also their ethers and esters, ketones such as acetone, methyl ethyl ketone, methyl isobutyl ketone or cyclohexanone, strongly polar solvents such as dimethyl sulphoxide, and also water.

Suitable solid carriers are:
for example, ammonium salts and ground natural minerals such as kaolins, clays, talc, chalk, quartz, attapulgite, montmorillonite or diatomaceous earth, and ground synthetic minerals, such as finely divided silica, alumina and silicates; suitable solid carriers for granules are: for example, crushed and fractionated natural rocks such as calcite, marble, pumice, sepiolite and dolomite, and also synthetic granules of inorganic and organic meals, and granules of organic material such as paper, sawdust, coconut shells, maize cobs and tobacco stalks; suitable emulsifiers and/or foam-formers are: for example, nonionic and anionic emulsifiers, such as polyoxyethylene fatty acid esters, polyoxyethylene fatty alcohol ethers, for example alkylaryl polyglycol ethers, alkylsulphonates, alkyl sulphates, arylsulphonates and also protein hydrolysates; suitable dispersants are nonionic and/or ionic substances, for example from the classes of the alcohol-POE and/or -POP ethers, acid and/or POP-POE esters, alkyl aryl and/or POP-POE ethers, fat and/or POP-POE adducts, POE- and/or POP-polyol derivatives, POE- and/or POP-sorbitan- or -sugar adducts, alkyl or aryl sulphates, alkyl- or arylsulphonates and alkyl or aryl phosphates or the corresponding PO-ether adducts. Furthermore, suitable oligo- or polymers, for example those derived from vinylic monomers, from acrylic acid, from EO and/or PO alone or in combination with, for example, (poly)alcohols or (poly)amines. It is also possible to employ lignin and its sulphonic acid derivatives, unmodified and modified celluloses, aromatic and/or aliphatic sulphonic acids and their adducts with formaldehyde.

Tackifiers such as carboxymethylcellulose and natural and synthetic polymers in the form of powders, granules or latices, such as gum arabic, polyvinyl alcohol and polyvinyl acetate, as well as natural phospholipids such as cephalins and lecithins, and synthetic phospholipids, can be used in the formulations.

It is possible to use colorants such as inorganic pigments, for example iron oxide, titanium oxide and Prussian Blue, and organic dyestuffs, such as alizarin dyestuffs, azo dyestuffs and metal phthalocyanine dyestuffs, and trace nutrients such as salts of iron, manganese, boron, copper, cobalt, molybdenum and zinc.

Other possible additives are perfumes, mineral or vegetable, optionally modified oils, waxes and nutrients (including trace nutrients), such as salts of iron, manganese, boron, copper, cobalt, molybdenum and zinc.

Stabilizers, such as low-temperature stabilizers, preservatives, antioxidants, light stabilizers or other agents which improve chemical and/or physical stability may also be present.

The formulations generally comprise between 0.01 and 98% by weight of active compound, preferably between 0.5 and 90%.

One skilled in the art will, of course, recognize that the formulation and mode of application of a toxicant may affect the activity of the material in a given application. Thus, for agricultural and general household pest use the present insecticidal compounds may be formulated as a granular of relatively large particle size (for example, 8/16 or 4/8 US Mesh), as water-soluble or water-dispersible granules, as powdery dusts, as wettable powders, as emulsifiable concentrates, as aqueous emulsions, as solutions, or as any of other known types of useful formulations, depending on the desired mode of application. It is to be understood that the amounts specified in this specification are intended to be approximate only, as if the word "about" were placed in front of the amounts specified.

These insecticidal compositions may be applied either as water-diluted sprays, or dusts, or granules to the areas in which suppression of insects is desired. These formulations may contain as little as 0.1%, 0.2% or 0.5% to as much as 95% or more by weight of active ingredient.

Dusts are free flowing admixtures of the active ingredient with finely divided solids such as talc, natural clays, kieselguhr, flours such as walnut shell and cottonseed flours, and other organic and inorganic solids which act as dispersants and carriers for the toxicant; these finely divided solids have an average particle size of less than about 50 microns. A typical dust formulation useful herein is one containing 1.0 part or less of the insecticidal compound and 99.0 parts of talc.

Wettable powders, also useful formulations for insecticides, are in the form of finely divided particles that disperse readily in water or other dispersant. The wettable powder is ultimately applied to the locus where insect control is needed either as a dry dust or as an emulsion in water or other liquid. Typical carriers for wettable powders include Fuller's earth, kaolin clays, silicas, and other highly absorbent, readily wet inorganic diluents. Wettable powders normally are prepared to contain about 5-80% of active ingredient, depending on the absorbency of the carrier, and usually also contain a small amount of a wetting, dispersing or emulsifying agent to facilitate dispersion. For example, a useful wettable powder formulation contains 80.0 parts of the insecticidal compound, 17.9 parts of Palmetto clay, and 1.0 part of sodium lignosulfonate and 0.3 part of sulfonated aliphatic polyester as wetting agents. Additional wetting agents and/or oils will frequently be added to a tank mix for to facilitate dispersion on the foliage of the plant.

Other useful formulations for insecticidal applications are emulsifiable concentrates (ECs) which are homogeneous liquid compositions dispersible in water or other dispersant, and may consist entirely of the insecticidal compound and a liquid or solid emulsifying agent, or may also contain a liquid carrier, such as xylene, heavy aromatic naphthas, isophorone, or other non-volatile organic solvents. For insecticidal application these concentrates are dispersed in water or other liquid carrier and normally applied as a spray to the area to be treated. The percentage by weight of the essential active ingredient may vary according to the manner in which the composition is to be applied, but in general comprises 0.5 to 95% of active ingredient by weight of the insecticidal composition.

Flowable formulations are similar to ECs, except that the active ingredient is suspended in a liquid carrier, generally water. Flowables, like ECs, may include a small amount of a surfactant, and will typically contain active ingredients in the range of 0.5 to 95%, frequently from 10 to 50%, by weight of the composition. For application, flowables may be diluted in water or other liquid vehicle, and are normally applied as a spray to the area to be treated.

Typical wetting, dispersing or emulsifying agents used in agricultural formulations include, but are not limited to, the alkyl and alkylaryl sulfonates and sulfates and their sodium salts; alkylaryl polyether alcohols; sulfated higher alcohols; polyethylene oxides; sulfonated animal and vegetable oils; sulfonated petroleum oils; fatty acid esters of polyhydric alcohols and the ethylene oxide addition products of such esters; and the addition product of long-chain mercaptans and ethylene oxide. Many other types of useful surface-active agents are available in commerce. Surface-active agents, when used, normally comprise 1 to 15% by weight of the composition.

Other useful formulations include suspensions of the active ingredient in a relatively non-volatile solvent such as water, corn oil, kerosene, propylene glycol, or other suitable solvents.

Still other useful formulations for insecticidal applications include simple solutions of the active ingredient in a solvent in which it is completely soluble at the desired concentration, such as acetone, alkylated naphthalenes, xylene, or other organic solvents. Granular formulations, wherein the toxicant is carried on relative coarse particles, are of particular utility for aerial distribution or for penetration of cover crop canopy. Pressurized sprays, typically aerosols wherein the active ingredient is dispersed in finely divided form as a result of vaporization of a low-boiling dispersant solvent carrier may also be used. Water-soluble or water-dispersible granules are free flowing, non-dusty, and readily water-soluble or water-miscible. In use by the farmer on the field, the granular formulations, emulsifiable concentrates, flowable concentrates, aqueous emulsions, solutions, etc., may be diluted with water to give a concentration of active ingredient in the range of say 0.1 % or 0.2% to 1.5% or 2%.

The active compound according to the invention can be used in its commercially available formulations and in the use forms, prepared from these formulations, as a mixture with other active compounds, such as insecticides, attractants, sterilizing agents, bactericides, acaricides, nematicides, fungicides, growth-regulating substances, herbicides, safeners, fertilizers or semiochemicals.

When used as insecticides, the active compounds according to the invention can furthermore be present in their commercially available formulations and in the use forms, prepared from these formulations, as a mixture with synergists. Synergists are compounds which increase the action of the active compounds, without it being necessary for the synergistic agent added to be active itself.

When used as insecticides, the active compounds according to the invention can furthermore be present in their commercially available formulations and in the use forms, prepared from these formulations, as a mixture with inhibitors which reduce degradation of the active compound after use in the environment of the plant, on the surface of parts of plants or in plant tissues.

The active compound content of the use forms prepared from the commercially available formulations can vary within wide limits. The active compound concentration of the use forms can be from 0.00000001 to 95% by weight of active compound, preferably between 0.00001 and 1% by weight.

The compounds are employed in a customary manner appropriate for the use forms.

All plants and plant parts can be treated in accordance with the invention. Plants are to be understood as meaning in the present context all plants and plant populations such as desired and undesired wild plants or crop plants (including naturally occurring crop plants). Crop plants can be plants which can be obtained by conventional plant breeding and optimization methods or by biotechnological and genetic engineering methods or by combinations of these methods, including the transgenic plants and including the plant cultivars protectable or not protectable by plant breeders' rights. Plant parts are to be understood as meaning all parts and organs of plants above and below the ground, such as shoot, leaf, flower and root, examples which may be mentioned being leaves, needles, stalks, stems, flowers, fruit bodies, fruits, seeds, roots, tubers and rhizomes. The plant parts also include harvested material, and vegetative and generative propagation material, for example cuttings, tubers, rhizomes, offshoots and seeds.

Treatment according to the invention of the plants and plant parts with the active compounds is carried out directly or by allowing the compounds to act on the surroundings, habitat or storage space by the customary treatment methods, for example by immersion, spraying, evaporation, fogging, scattering, painting on, injection and, in the case of propagation material, in particular in the case of seeds, also by applying one or more coats.

Treatment according to the invention of the plants and plant parts with the active compound combinations is carried out directly or by allowing the compounds to act on the surroundings, habitat or storage space by the customary treatment methods, for example by immersion, spraying, evaporation, fogging, scattering, painting on, and, in the case of propagation material, in particular in the case of seeds, also by applying one or more coats.

### Seed Treatment

Besides the treatment of plants or plant parts other than seeds, the methods and compositions of the invention are particularly suitable for the treatment of seeds. A large part of the damage caused by pests and pathogens on cultigens occurs by infestation of the seed during storage and after sowing the seed in the ground as well as during and immediately after germination of the plants. This phase is especially critical since the roots and shoots of the growing plant are particularly sensitive and even a small amount of damage can lead to withering of the whole plant. There is therefore considerable interest in protecting the seed and the germinating plant by the use of suitable agents.

The control of pests and pathogens by treatment of the seeds of plants has been known for a considerable time and is the object of continuous improvement. However, there are a number of problems in the treatment of seed that cannot always be satisfactorily solved. Therefore it is worthwhile to develop methods for the protection of seeds and germinating plants which makes the additional application of plant protection agents after seeding or after germination of the plants superfluous. It is further worthwhile to optimize the amount of the applied active material such that the seed and the germinating plants are protected against infestation by pests as best as possible without the plants themselves being damaged by the active compound applied. In particular, methods for the treatment seed should also take into account the intrinsic insecticidal and fungicidal properties of transgenic plants in order to achieve optimal protection of the seed and germinating plants with a minimal expenditure of plant protection agents.

The present invention relates therefore especially to a method for the protection of seed and germinating plants from infestation with pests and pathogens in that the seed is treated with a combination of the invention.

The invention comprises a procedure in which the seed the treated at the same time with components Fluopyram and optionally furher active ingredients. It further comprises a method in which the seed is treated with Fluopyram and optional furher active ingredients separately.

The invention also comprises a seed, which has been treated with Fluopyram and optional furher active ingredients at the same time or separately, and which still contains an effective amount of these active ingredients. For the latter seed, the active ingredients can be applied in separate layers. These layers can optionally be separated by an additional layer that may or may not contain an active ingredient.

The time interval between the application of different layers of the style compounds is in general not critical.

In addition the invention relates also to the use of the combination of the invention for the treatment seed for protection of the seed and the germinating plants from pests. Furthermore the invention relates to seed which was treated with an agent of the invention for protection from pests.

One of the advantages of the invention is because of the special systemic properties of the agents of the invention treatment with these agents protects not only the seed itself from pests but also the plants emerging after sprouting. In this way the direct treatment of the culture at the time of sowing or shortly thereafter can be omitted.

The agents of the invention are suitable for the protection of seed of plant varieties of all types as already described which are used in agriculture, in greenhouses, in forestry, in garden construction or in vineyards. In particular, this concerns seed of maize, peanut, canola, rape, poppy, olive, coconut, cacao, soy cotton, beet, (e.g. sugar beet and feed beet), rice, millet, wheat, barley, oats, rye, sunflower, sugar cane or tobacco. The agents of the invention are also suitable for the treatment of the seed of fruit plants and vegetables as previously described. Particular importance is attached to the treatment of the seed of maize, soy, cotton, wheat and canola or rape. Thus, for example, the combination of number (1) is particularly suitable for the treatment of maize seed.

As already described, the treatment of transgenic seed with an agent of the invention is of particular importance. This concerns the seeds of plants which generally contain at least one heterologous gene that controls the expression of a polypeptide with special insecticidal properties. The heterologous gene in transgenic seed can originate from microorganisms such as Bacillus, Rhizobium, Pseudomonas, Serratia, Trichoderma, Clavibacter, Glomus or Gliocladium. The present invention is particularly suitable for the treatment of transgenic seed that contains at least one heterologous gene that originates from Bacillus sp. and whose gene product exhibits activity against the European corn borer and/or western corn rootworm. Particularly preferred is a heterologous gene that originates from Bacillus thuringiensis.

Within the context of the present invention the agent of the invention is applied to the seed alone or in a suitable formulation. Preferably the seed is handled in a state in which it is so stable, that no damage occurs during treatment. In general treatment of the seed can be carried out at any time between harvest and sowing. Normally seed is used that was separated from the plant and has been freed of spadix, husks, stalks, pods, wool or fruit flesh. Use of seed that was harvested, purified, and dried to moisture content of below 15 % w/w. Alternatively, seed treated with water after drying and then dried again can also be used.

In general care must be taken during the treatment of the seed that the amount of the agent of the invention and/or further additive applied to the seed is so chosen that the germination of the seed is not impaired and the emerging plant is not damaged. This is to be noted above all with active compounds which can show phytotoxic effects when applied in certain amounts.

The compositions of the invention can be applied directly, that is without containing additional components and without being diluted. It is normally preferred to apply the agent to the seed in the form of a suitable formulation. Suitable formulations and methods for seed treatment are known to the person skilled in the art and are described, for example, in the following documents: US 4,272,417 A, US 4,245,432 A, US 4,808,430 A, US 5,876,739 A, US 2003/0176428 A1, WO 2002/080675 A1, WO 2002/028186 A2.

Compositions, which are especially useful for seed treatment, are e.g.:
A Soluble concentrates (SL, LS)
D Emulsions (EW, EO, ES)
E Suspensions (SC, OD, FS)
F Water-dispersible granules and water-soluble granules (WG, SG)
G Water-dispersible powders and water-soluble powders (WP, SP, WS)
H Gel-Formulations (GF)
I Dustable powders (DP, DS)

Conventional seed treatment formulations include for example flowable concentrates FS, solutions LS, powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter. Preferred are FS formulations.

In the treatment of seed, the application rates of the inventive combination are generally from 0.1 to 10 kg per 100 kg of seed. The separate or joint application of the compounds I and II or of the combinations of the compounds I and II is carried out by spraying or dusting the seeds, the seedlings, the plants or the soils before or after sowing of the plants or before or after emergence of the plants.

The invention also relates to the propagation products of plants, and especially the seed comprising, that is, coated with and/or containing, a combination as defined above or a composition containing the combination of two or more active ingredients or a combination of two or more compositions each providing one of the active ingredients. The seed comprises the inventive combinations in an amount of from 0.1 g to 10 kg per 100 kg of seed.

The composition comprising a combination of pesticides 45 can be applied "neat",that is, without any diluting or additional components present. However, the composition is typically applied to the seeds in the form of a pesticide formulation. This formulation may contain one or more other desirable components including but not limited to 50 liquid diluents, binders to serve as a matrix for the pesticide, fillers for protecting the seeds during stress conditions, and plasticizers to improve flexibility, adhesion and/or spreadability of the coating. In addition, for oily pesticide formulations containing little or no filler, it may be desirable to add 55 to the formulation drying agents such as calcium carbonate, kaolin or bentonite clay, perlite, diatomaceous earth or any other adsorbent material. Use of such components in seed treatments is known in the art. See, e.g., U.S. Pat. No. 5,876,739. The skilled artisan can readily select desirable 60 components to use in the pesticide formulation depending on the seed type to be treated and the particular pesticide that is selected. In addition, readily available commercial formulations of known pesticides may be used, as demonstrated in the examples below.

The seeds may also be treated with one or more of the following ingredients: other pesticides, including compounds which act only below the ground; fungicides, such as captan, thiram, metalxyl, fhidioxonil, oxadixyl, and isomers of each of those materials, and the like; herbicides, including compounds selected from acetamides, triazines, dinitroanilines, glycerol ethers, pyridazinones, uracils, phenoxys, ureas, and benzoic acids; herbicidal safeners such as benzoxazine, benzhydryl derivatives, N,N-diallyl dichloroacetamide, various dihaloacyl, oxazolidinyl and thiazolidinyl compounds, ethanone, naphthalic anhydride compounds, and oxime derivatives; fertilizers; and biocontrol agents such as naturally-occurring or recombinant bacteria and fungi from the genera Rhizobium, Bacillus, Pseudomonas, Serratia, Trichoderma, Glomus, Gliocladium and mycorrhizal fungi. These ingredients may be added as a separate layer on the seed or alternatively may be added as part of the pesticide composition.

Preferably, the amount of the novel composition or other ingredients used in the seed treatment should not inhibit generation of the seed, or cause phytotoxic damage to the seed.

The composition of the present invention can be in the form of a suspension; emulsion; slurry of particles in an aqueous medium (e.g., water); wettable powder; wettable granules (dry flowable); and dry granules. If formulated as a suspension or slurry, the concentration of the active ingredient in the formulation is preferably about 0.5% to about 99% by weight (w/w), preferably 5-40%.

As mentioned above, other conventional inactive or inert ingredients can be incorporated into the formulation. Such inert ingredients include but are not limited to: conventional sticking agents, dispersing agents such as mefhylcellulose (Methocel A15LV or Methocel A15C, for example, serve as combined dispersant/sticking agents for use in seed treatments), polyvinyl alcohol (e.g., Elvanol 51-05), lecithin (e.g., Yelkinol P), polymeric dispersants (e.g., polyvinylpyrrolidone/vinyl acetate PVP/VA S-630), thickeners (e.g., clay thickeners such as Van Gel B to improve viscosity and reduce settling of particle suspensions), emulsion stabilizers, surfactants, antifreeze compounds (e.g., urea), dyes, colorants, and the like. Further inert ingredients useful in the present invention can be found in McCutcheon's, vol. 1, "Emulsifiers and Detergents" MC Publishing Company, Glen Rock, N.J., U.S.A., 1996. Additional inert ingredients useful in the present invention can be found in McCutcheon's, vol.2, "FunctionalMaterials," MC Publishing Company, Glen Rock, N.J., U.S.A., 1996.

The pesticides, compositions of pesticide combinations, and formulations of the present invention can be applied to seeds by any standard seed treatment methodology, including but not limited to mixing in a container (e.g., a bottle or bag), mechanical application, tumbling, spraying, and immersion. Any conventional active or inert material can be used for contacting seeds with pesticides according to the present invention, such as conventional film-coating materials including but not limited to water-based film coating materials such as Sepiret (Seppic, Inc., Fairfield, N.J.) and Opacoat (Berwind Pharm. Services, Westpoint, Pa.).

*Seed coating:* The subject combination of pesticides can be applied to a seed as a component of a seed coating. Seed coating methods and compositions that are known in the art are useful when they are modified by the addition of one of the embodiments of the combination of pesticides of the present invention. Such coating methods and apparatus for their application are disclosed in, for example, U.S. Pat. Nos. 5,918,413, 5,891,246, 5,554,445, 5,389,399, 5,107,787, 5,080,925, 4,759,945 and 4,465,017. Seed coating compositions are disclosed, for example, in U.S. Pat. Nos. 5,939,356, 5,882,713, 5,876,739, 5,849,320, 5,834,447, 5,791,084, 5,661,103, 5,622,003, 5,580,544, 5,328,942, 5,300,127, 4,735,015, 4,634,587, 4,383,391, 4,372,080, 4,339,456, 4,272,417 and 4,245,432, among others. Useful seed coatings contain one or more binders and at least one of the subject combinations of pesticides.

Useful seed coatings contain one or more binders and at least one of the subject combinations of pesticides.

Binders that are useful in the present invention preferably comprise an adhesive polymer that may be natural or synthetic and is without phytotoxic effect on the seed to be coated. The binder may be selected from polyvinyl acetates; polyvinyl acetate copolymers; polyvinyl alcohols; polyvinyl alcohol copolymers; celluloses, including ethylcelluloses, methylcelluloses, hydroxymethylcelluloses, hydroxypropy-lcelluloses and carboxymethylcellulose; polyvinylpyroh-dones; polysaccharides, including starch, modified starch, dextrins, maltodextrins, alginate and chitosans; fats; oils; proteins, including gelatin and zeins; gum arabics; shellacs; vinylidene chloride and vinylidene chloride copolymers; calcium lignosulfonates; acrylic copolymers; polyvinylacrylates; polyethylene oxide; acrylamide polymers and copolymers; polyhydroxyethyl acrylate, mefhylacrylamide monomers; and polychloroprene.

It is preferred that the binder be selected so that it can serve as a matrix for the subject combination of pesticides. While the binders disclosed above may all be useful as a matrix, the specific binder will depend upon the properties of the combination of pesticides. The term "matrix", as used herein, means a continuous solid phase of one or more binder compounds throughout which is distributed as a discontinuous phase one or more of the subject combinations of pesticides. Optionally, a filler and/or other components can also be present in the matrix. The term matrix is to be understood to include what may be viewed as a matrix system, a reservoir system or a microencapsulated system. In general, a matrix system consists of a combination of pesticides of the present invention and filler uniformly dispersed within a polymer, while a reservoir system consists of a separate phase comprising the subject combination of pesticides, that is physically dispersed within a surrounding, rate-limiting, polymeric phase. Microencapsulation includes the coating of small particles or droplets of liquid, but also to dispersions in a solid matrix.

The amount of binder in the coating can vary, but will be in the range of about 0.01 to about 25% of the weight of the seed, more preferably from about 0.05 to about 15%, and even more preferably from about 0.1% to about 10%.

As mentioned above, the matrix can optionally include a filler. The filler can be an absorbent or an inert filler, such as are known in the art, and may include wood flours, clays, activated carbon, sugars, diatomaceous earth, cereal flours, fine-grain inorganic solids, calcium carbonate, and the like. Clays and inorganic solids which may be used include calcium bentonite, kaolin, china clay, talc, perlite, mica, vermiculite, silicas, quartz powder, montmoriUonite and mixtures thereof. Sugars which may be useful include dextrin and maltodextrin. Cereal flours include wheat flour, oat flour and barley flour.

The filler is selected so that it will provide a proper microclimate for the seed, for example the filler is used to increase the loading rate of the active ingredients and to adjust the control-release of the active ingredients. The filler can aid in the production or process of coating the seed. The amount of filler can vary, but generally the weight of the filler components will be in the range of about 0.05 to about 75% of the seed weight, more preferably about 0.1 to about 50%, and even more preferably about 0.5% to 15%.

The pesticides that are useful in the coating are those combinations of pesticides that are described herein. The amount of pesticide that is included in the coating will vary depending upon the type of seed and the type of active ingredients, but the coating will contain an amount of the combination of pesticides that is pesticidally effective. When insects are the target pest, that amount will be an amount of the combination of insecticides that is insecticidally effective. As used herein, an insecticidally effective amount means that amount of insecticide that will kill insect pests in the larvae or pupal state of growth, or will consistently reduce or retard the amount of damage produced by insect pests. In general, the amount of pesticide in the coating will range from about 0.005 to about 50% of the weight of the seed. A more preferred range for the pesticide is from about 0.01 to about 40%; more preferred is from about 0.05 to about 20%.

The exact amount of the combination of pesticides that is included in the coating is easily determined by one of skill in the art and will vary depending upon the size of the seed to be coated. The pesticides of the coating must not inhibit germination of the seed and should be efficacious in protecting the seed and/or the plant during that time in the target insect's life cycle in which it causes injury to the seed or plant. In general, the coating will be efficacious for approximately 0 to 120 days after sowing.

The coating is particularly effective in accommodating high pesticidal loads, as can be required to treat typically refractory pests, such as corn root worm, while at the same time preventing unacceptable phytotoxicity due to the increased pesticidal load.

Optionally, a plasticizer can be used in the coating formulation. Plasticizers are typically used to make the film that is formed by the coating layer more flexible, to improve adhesion and spreadability, and to improve the speed of processing. Improved film flexibility is important to minimize chipping, breakage or flaking during storage, handling or sowing processes. Many plasticizers may be used. However, useful plasticizers include polyethylene glycol, glycerol, butylbenzylphthalate, glycol benzoates and related compounds. The range of plasticizer in the coating layer will be in the range of from bout 0.1 to about 20% by weight.

When the combination of pesticides used in the coating is an oily type formulation and little or no filler is present, it may be useful to hasten the drying process by drying the formulation. This optional step may be accomplished by means will known in the art and can include the addition of calcium carbonate, kaolin or bentonite clay, perlite, diatomaceous earth, or any absorbent material that is added preferably concurrently with the pesticidal coating layer to absorb the oil or excess moisture. The amount of calcium carbonate or related compounds necessary to effectively provide a dry coating will be in the range of about 0.5 to about 10% of the weight of the seed.

The coatings formed with the combination of pesticides are capable of effecting a slow rate of release of the pesticide by diffusion or movement through the matrix to the surrounding medium.

The coating can be applied to almost any crop seed that is described herein, including cereals, vegetables, ornamentals and fruits.

In addition to the coating layer, the seed may be treated with one or more of the following ingredients: other pesticides including fungicides and herbicides; herbicidal safeners; fertilizers and/or biocontrol agents. These ingredients may be added as a separate layer or alternatively may be added in the pesticidal coating layer.

The pesticide formulation may be applied to the seeds using conventional coating techniques and machines, such as fluidized bed techniques, the roller mill method, rotostatic seed treaters, and drum coaters. Other methods, such as spouted beds may also be useful. The seeds may be presized 5 before coating. After coating, the seeds are typically dried and then transferred to a sizing machine for sizing. Such procedures are known in the art.

The pesticide-treated seeds may also be enveloped with a film overcoating to protect the pesticide coating. Such overcoatings are known in the art and may be applied using conventional fluidized bed and drum film coating techniques.

In another embodiment of the present invention, a pesticide can be introduced onto or into a seed by use of solid matrix priming. For example, a quantity of the pesticide can be mixed with a solid matrix material and then the seed can be placed into contact with the solid matrix material for a period to allow the pesticide to be introduced to the seed. The seed can then optionally be separated from the solid matrix material and stored or used, or the mixture of solid matrix material plus seed can be stored or planted directly. Solid matrix materials which are useful in the present invention include polyacrylamide, starch, clay, silica, alumina, soil, sand, polyurea, poly aery late, or any other material capable of absorbing or adsorbing the pesticide for a time and releasing that pesticide into or onto the seed. It is useful to make sure that the pesticide and the solid matrix material are compatible with each other. For example, the solid matrix material should be chosen so that it can release the pesticide at a reasonable rate, for example over a period of minutes, hours, or days.

The present invention further embodies inhibition as another method of treating seed with the pesticide. For example, plant seed can be combined for a period of time with a solution comprising from about 1% by weight to about 75% by weight of the pesticide in a solvent such as water. Preferably the concentration of the solution is from about 5% by weight to about 50% by weight, more preferably from about 10% by weight to about 25% by weight. During the period that the seed is combined with the solution, the seed takes up (imbibes) a portion of the pesticide. Optionally, the mixture of plant seed and solution can be agitated, for example by shaking, rolling, tumbling, or other means. After inhibition, the seed can be separated from the solution and optionally dried, for example by patting or air drying.

In yet another embodiment, a powdered pesticide can be mixed directly with seed. Optionally, a sticking agent can be used to adhere the powder to the seed surface. For example, a quantity of seed can be mixed with a sticking agent and optionally agitated to encourage uniform coating of the seed with the sticking agent. The seed coated with the sticking agent can then be mixed with the powdered pesticide. The mixture can be agitated, for example by tumbling, to encourage contact of the sticking agent with the powdered pesticide, thereby causing the powdered pesticide to stick to the seed.

The present invention also provides a seed that has been treated by the method described above. The treated seeds of the present invention can be used for the propagation of plants in the same manner as conventional treated seed. The treated seeds can be stored, handled, sowed and tilled in the same manner as any other pesticide treated seed. Appropriate safety measures should be taken to limit contact of the treated seed with humans, food or feed materials, water and birds and wild or domestic animals.

## Claims

1. A method for increasing the production potential of plants and/or controlling pests in plants with at least one transgenic modification related to yield increase as compared to a corresponding wild-type plant, comprising treating the location where the plant with at least one transgenic modification is growing or is expected to grow and/or the transgenic plant with at least one transgenic modification or propagation material of the plant with at least one transgenic modification with an effective amount of a chemical composition comprising Fluopyram.

2. The method of claim 1, wherein the transgenic plant
a. is selected from the plants listed in Table A: A-1 to A-131 or
b. is selected from the plants listed in Table B: B-1 to B-85, or
c. comprises one or more transgenic events selected from the transgenic events listed in Table A from A-1 to A-131 or in Table B from B-1 to B-85, or
d. displays a trait based one or several transgenic events as listed in Table C from C-1 to C-11, or

3. The method of claims 1 or 2, wherein the pests are arachnids, insects, or nematodes.

4. The method of any of the claims 1 to 3, wherein strains of the pests are targeted that are at least partially resistant or tolerant to the transgenic events that confer resistance of the plant against the wildtype or sensitive strains of the foresaid pest.

5. The method of any of the claims 1 to 4, wherein the plants are selected from: maize, soya bean, cotton, tobacco, rice, potato and sugar beet.

6. The method of any one of the claims 1 to 5, wherein an additional active ingredient is used with Fluopyram, wherein this active ingredient is selected from the group consisting of acephate, chlorpyrifos, diazinon, dichlorvos, dimethoate, fenitrothion, methamidophos, methidathion, methyl-parathion, monocrotophos, phorate, profenofos, terbufos, aldicarb, carbaryl, carbofuran, carbosulfan, methomyl, thiodicarb, bifenthrin, cyfluthrin, cypermethrin, alpha-cypermethrin, zeta-cypermethrin, deltamethrin, esfenvalerate, lambda-cyhalothrin, permethrin, tefluthrin, diflubenzuron, flufenoxuron, lufenuron, teflubenzuron, spirotetramat; clothianidin, dinotefuran, imidacloprid, thiamethoxam, acetamiprid, thiacloprid; endosulfan, ethiprole, abamectin, emamectin, spinosad, spinetoram.

7. The method of any one of the claims 1 to 5, wherein an additional active ingredient is used with Fluopyram, wherein this active ingredient is selected from the group consistinig of azoxystrobin, dimoxystrobin, kresoxim-methyl, orysastrobin, pyraclostrobin, trifloxystrobin, bixafen, boscalid, isopyrazam, metalaxyl, penthiopyrad, 3-difluoromethyl-1-methyl-1 H-pyrazole-4-carboxylic acid (2',4',5'-trifluorobiphenyl-2-yl)-amide, N-(2-bicyclopropyl-2-yl-phenyl)-3-difluoromethyl-1-methyl-1H-pyrazole-4-carboxylic acid amide, dimethomorph, fluopicolide, difenoconazole, epoxiconazole, fluquinconazole, flusilazole, flutriafol, metconazol, myclobutanil, propiconazole, prothioconazole, tebuconazole, tetraconazole, triticonazole, prochloraz, carbendazim, fluazinam, cyprodinil, pyrimethanil, fludioxonil, dodemorph, fenpropimorph, tridemorph, fenpropidin, iprodione, vinclozolin, famoxadone, probenazole, captan, folpet, 5-ethyl-6-octyl-[1,2,4]triazolo[1,5-a]pyrimidine-7-ylamine, mancozeb, maneb, metiram, thiram, dithianon, fosetyl, fosetyl-aluminium, chlorothalonil, thiophanate methyl, cymoxanil, metrafenone, spiroxamine, bixafen, N-(3',4',5'-trifluorobiphenyl-2-yl)- 3-difluoromethyl- 1 -methyl-1 H-pyrazole-4-carboxamide, N-[2-(4'-trifluoromethylthio)-biphenyl]-3-difluoromethyl-1 -methyl-1 H-pyrazole-4-carboxamide,N-[2-(1,3-dimethylbutyl)-phenyl]- 1,3-dimethyl-5-fluoro-1 H-pyrazole-4-carboxamide, N-(2-bicyclopropyl-2-yl-phenyl)-3-difluoromethyl-1-methyl-1H-pyrazole-4-carboxamide,N-(cis-2-bicyclopropyl-2-yl-phenyl)-3-difluoromethyl-1 -methyl-1 H-pyrazole-4-carboxamide, N-(trans-2-bicyclopropyl-2-yl-phenyl)-3-difluoromethyl-1-methyl-1 H-pyrazole-4-carboxamide , N-[1,2,3,4-tetrahydro-9-(1 -methylethyl)-1,4-methanonaphthalen-5-yl]-3-(difluoromethyl)-1-methyl-1 H-pyrazole-4-carboxamide.

8. The method to any of the claims 1 to 8, wherein a seed is treated.

9. The method to any of the claims 1 to 8, for increasing the yield of the plant.

10. The method to any of the claims 1 to 8, for increasing the tolerance of the plant against abiotic stress.
